(19) Europäisches Patentamt

European Patent Office

Office européen des brevets

(11) Publication number: **0 327 626 B1**

(12) **EUROPEAN PATENT SPECIFICATION**

(45) Date of publication of patent specification: **21.04.93**

(51) Int. Cl.⁵: **C12N 15/00**, C12N 15/86, C12N 7/00, C12P 21/02

(21) Application number: **88906974.6**

(22) Date of filing: **11.08.88**

(86) International application number:
**PCT/GB88/00663**

(87) International publication number:
**WO 89/01518 (23.02.89 89/05)**

Consolidated with 88307452.8/0305087
(European application No./publication No.) by
decision dated 09.04.91.

(54) EXPRESSION VECTORS FOR THE SYNTHESIS OF PROTEINS AND PLASMID REPLICONS AND SEOUENCE CASSETTES FOR USE IN CONSTRUCTING SUCH VECTORS.

(30) Priority: **12.08.87 GB 8719108**
**12.07.88 GB 8816084**

(43) Date of publication of application:
**16.08.89 Bulletin 89/33**

(45) Publication of the grant of the patent:
**21.04.93 Bulletin 93/16**

(84) Designated Contracting States:
**AT BE CH DE ES FR GB GR IT LI LU NL SE**

(56) References cited:
**WO-A-88/00971**

**Vaccines 86, New Approaches Immun., 1986, Cold Spring Harbor Laboratory, (New York, US), S. Chakrabarti et al.: "New vaccinia virus exression vector", pp. 289-292, s. whole article, esp. fig. 2**

(73) Proprietor: **NATURAL ENVIRONMENT RE-SEARCH COUNCIL**
**Polaris House North Star Avenue**
**Swindon SN2 1EU(GB)**

(72) Inventor: **BISHOP, David, Hugh, Langler**
**8 Carey Close**
**Oxford(GB)**
Inventor: **EMERY, Vincent, Clive**
**Flat 2 31 Hillfield Road**
**London NW6 1OD(GB)**

(74) Representative: **Ritter, Stephen David et al**
**Mathys & Squire 10 Fleet Street**
**London EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

Chemical Abstracts, vol. 105, no. 25, December 1986, (Columbus, Ohio, US), A.D. Al'tshtein et al.: "Vaccinia virus double recombinant, expressing the hepatitis B virus surface antigen and herpes simplex virus thymidine kinase", s. p. 177, abstract 219836x, & Dokl. Adad. Nauk SSSSR 1986, 289 (6), 1493-6

Protein Engineering, vol. 1, no. 4, 15 September 1987, IRL Press Ltd, (Oxford, GB), V.C. Emery et al.: "The development of multiple expression vectors for high level synthesis of eukaryotic proteins: expression of LCMV-N and AcNPV polyhedrin protein by a recombinant baculovirus", pp. 359-366

The Journal of General Virology, vol. 68, no. 5, May 1987, SGM, (GB), Y Matsuura et al.: "Baculovirus expression vectors: the requirements for high level expression of proteins, incl. glycoproteins", pp. 1233-1250

Vaccines 87, 1987, Cold Spring Harbor Laboratory, (New York, US), M.A. Cochran et al.: "Use of baculovirus recombinants as a general method for the production of subunit vaccines", pp. 384-388, s. p. 384 and fig. 1

**Description**

This invention relates to expression vectors for the expression of proteins, particularly eukaryotic proteins, and to plasmid replicons and sequence cassettes useful in constructing such vectors. The invention particularly relates to improved expression vectors useful for expressing selected proteins in insects and insect cells.

To address some of the more challenging aspects of molecular biology, for example, the synthesis of products involving multiple proteins, the investigation of the factors involved in heterologous protein-protein interactions and the synthesis of products involving consecutive enzymatic processes, new expression systems that produce several gene products simultaneously are required (so-called "polygenic expression"), including ones that can reproducibly make different proteins at different predetermined levels.

This particularly applies to hepatitis B surface and core antigens. That is to say it would be particularly desirable in order to produce diagnostic reagents and vaccines to be able to co-produce Hepatitis B surface and core antigens. Hepatitis B is a disease of major significance world-wide. Infected individuals may experience only an acute infection, or may develop a long term infection that is associated with liver cirrhosis and hepatocellular carcinoma, chronic active HB, chronic persistent HB and chronic lobular HB. In certain regions of the world, particularly, but not exclusively in South-east Asia and Africa, persistent infections may account for up to 15% of the population with as many as 40% of those individuals liable to die from one or another form of the disease. Estimates have been made that world-wide up to 300 million people are carriers of the disease (i.e., persistently infected with the virus). Plasma and rDNA derived preparations that contain the HBsAg have been used to vaccinate people against the disease.

A difficulty which often arises when attempting to produce new constructs which combine in a single expression vector genes coding for different proteins is that it can be difficult to select recombinant entities containing both desired genes in functional form or in a form in which the gene products can be expressed in predetermined quantities. Further the constructs obtained can be genetically unstable, that is to say naturally occurring recombinational events can result in loss of one or more of the introduced genes from the vectors or from the plasmid replicons used to produce them.

The production of eukaryotic proteins using expression vectors derived from the *Autographia californica* nuclear polyhedrosis virus has been described, e.g. by Smith *et al.*, "Modification and Secretion of Human Interleukin-2 Produced in Insect Cells by a Baculovirus Expression Vector", Proc. Natl. Acad. Sci. USA, *82* No. 24 (1985) 8404-8408 and by Matsuura *et al.*, "Baculovirus Expression Vectors: The Requirement for High Level Expression of Proteins, Including Glycoproteins", J. Gen. Virol. (1987) *68*, 1233-1250. However in available baculovirus expression systems, no ready means exists which enables the expression of more than one gene product simultaneously in a reproducible manner. Further, available expression vectors lack the infective capability of the native virus. This is believed to be due to the fact that native virus produces, during late stages of infection, so-called inclusion bodies formed from a protein, polyhedrin, which is encoded in the native viral genome. Recombinant expression vectors designed to express desired proteins in the baculovirus expression system hitherto have sought to produce the desired protein in favour of polyhedrin. The expression vectors cannot readily be produced in the form of inclusion bodies - a form which is known to be particularly infective of insect larvae.

Chakrabarti *et al.* in an article entitled "New Vaccinia Virus Expression Vector" (Vaccines 86, Publn Cold Spring Harbour Laboratory and Chem. Abst. *105* (1986) 166241r) described a coexpression vector in the vaccinia system that coexpresses the β-Gal gene of *E.coli* and a second foreign gene. Expression of the β-Gal gene is used to screen for recombinant vaccinia virus. However no procedures are described enabling the reproducible expression of a plurality of eukaryotic proteins.

The present invention provides plasmid replicons, sequence cassettes and expression vectors in which the deficiencies of the prior art are avoided or substantially diminished.

Particularly multiple expression vectors that make foreign gene products as well as polyhedrin protein have now been developed. Since such recombinant viruses are occluded they are highly infectious in caterpillar hosts that are permissive for the parent virus, allowing the production of the foreign gene product in a cost effective manner. Multiple expression vectors have also been developed in which two foreign gene products are made by a recombinant virus (e.g., HBcAg and MBsAg), as well as occluded recombinant viruses that make MBsAg, and single expression vectors that make high levels of HBcAG, or HBpcAg.

According to one aspect of the present invention there are provided plasmid replicons for use in introducing a plurality of genes into an expression vector, comprising double-stranded DNA having

(a) one or more sequences allowing the replicon to be reproduced in a bacterial host,
(b) first and second sequences which are adapted to permit an intervening sequence located between said first and second sequences to be introduced into an expression vector, and

(c) an intervening sequence located between said first and second sequences.

The plasmid replicons are characterised in that the intervening sequence comprises first and second polypeptide expression sequences (PESs) wherein each PES includes (i) a transcriptional promotor (ii) a unique restriction site for introduction of a gene which is native or foreign to the expression vector and (iii) a transcriptional termination site. Preferably each PES includes a different unique restriction site for introduction of a gene which is native or foreign to the expression vector.

The term "PES" will be used hereafter to denote "polypeptide expression sequence".

In order to reduce the possibility of recombinational elimination of desired introduced genes, the first and second PESs are advantageously arranged in the opposite sense to one another on separate strands of the DNA.

Alternatively the first and second PESs may be arranged in the same sense on the same strand of DNA. With this construction it is particularly preferred that a selectable gene or an essential functional gene for the expression vector is located between the two PESs to select against derivatives that have eliminated one or other PES by, e.g. natural recombination.

In order to prepare the plasmid replicons for introducing a plurality of genes into an expression vector capable of transfecting a susceptible insect or insect cell, genes which are native or foreign to the expression vector may be introduced at the sites (ii) of the PESs. The resulting "loaded" plasmid replicons, i.e. plasmid replicons loaded with desired genes for introduction into an expression vector form a further aspect of the present invention. Such "loaded" plasmid replicons comprise double stranded DNA having

(a) one or more sequences allowing the replicon to be reproduced in a bacterial host,
(b) first and second sequences which are adapted to permit an intervening sequence located between said first and second sequences to be introduced into an expression vector, and
(c) an intervening sequence located between said first and second sequences,

and are characterised in that the intervening sequence comprises first and second polypeptide expression sequences (PESs) wherein each PES includes (i) a transcriptional promotor (ii) a gene which is native or foreign to the expression vector and (iii) a transcriptional termination site. The transcriptional termination site can be native or foreign to the expression vector.

Advantageously, the plasmid replicons according to the invention are used to construct vectors capable of transfecting susceptible insect cells. Examples include vectors derived from viruses, the wild type or derivatives of which are capable of infecting such cells. In such cases one or more of the genes present in the PESs may code for viral protein normally expressed during infection of the cells by wild type virus.

Alternatively or additionally the gene introduced into at least one the PESs may represent a selectable foreign protein or a normal gene product of the vector.

The production of one or more selectable or normal gene products may then be used as marker(s) for the detection of desired recombinant plasmid replicons or expression vectors. Further, at least one of said promoters in the PESs may be a promotor for a viral protein normally expressed during infection of the cells by wild type virus.

The plasmid replicons according to the invention (both loaded and unloaded) may be constructed from so-called "sequence cassettes" which themselves form a further aspect of the invention. These sequence cassettes comprise recombinant DNA molecules having an intervening sequence which comprises first and second polypeptide expression sequences (PESs) wherein each PES is as defined above either for the loaded or unloaded plasmid replicons, flanked by arms which are homologous with sequences of an expression vector, such that when inserted in the vector, no essential functional genes of the expression vector are lost.

In a first preferred construction of sequence cassette, the arms are homologous to sequences of the vector genome, which sequences are arranged so as to allow the intervening sequences to be introduced without loss of vector DNA sequences.

In a second preferred construction the arms are homologous to sequences of the vector genome, which sequences are arranged so as to allow the intervening sequences to be introduced with replacement of inessential vector DNA sequences by intervening sequences of the sequence cassette.

In a third preferred construction the arms are homologous to sequences of the vector genome, which sequences are arranged so as to allow the intervening sequences to be introduced so as to replace intergenic or non-regulatory regions of the vector genome.

Sequence cassettes, according to the invention may be constructed in sets wherein the homologous regions of each cassette of the set are homologous to different regions of the vector genome, allowing a plurality of pairs of PESs to be introduced into the vector genome, with each pair being located at a separate location.

Expression vectors, suitable for transfecting insect cells may be produced from the plasmid replicons defined above. These expression vectors are themselves novel and form a further aspect of the invention and have an insert adapted to direct synthesis in the cell of at least one polypeptide not normally encoded by nuclear DNA of the cell, said insert comprising double-stranded DNA having an intervening sequence located between said first and second sequences. The expression vectors are characterised in that the intervening sequence comprises first and second polypeptide expression sequences (PESs) wherein each PES includes (i) a transcriptional promotor (ii) a gene which is native or foreign to the expression vector and (iii) a transcriptional termination site. The transcriptional termination site can be native or foreign to the expression vector.

These expression vectors may be derived from a viral entity, the wild type of which is infectious of insect cells. In this case one of said structural genes may advantageously code for a viral protein normally expressed during infection of the cells by wild type virus. In these preferred expression vectors at least one of said promotors can be a promotor for a viral protein normally expressed during infection of the cells by wild type virus.

Specific examples of expression vectors according to the invention derived from viruses are ones wherein the viral entity is a baculovirus. In this case one of said structural genes codes can conveniently code for AcNPV polyhedrin protein and at least one of said promotors can be the promotor of the polyhedrin gene of *Autographa californica* nuclear polyhedrosis virus.

Recombinant plasmid replicons and expression vectors according to the invention may comprise only a single pair of said PESs or they may include a plurality of such pairs. Thus they may be derived from only a single cassette of the kind described above or they may be derived from a plurality of such cassettes.

In the latter case it is preferable that there are one or more essential functional genes or selectable genes located between the PESs with regulatory elements and sequences of these essential or selectable genes being distinguished from those of the PESs. By constructing plasmid replicons and expression vectors in this way it is possible to reduce or minimise the likelihood of gene elimination by recombination.

The plasmid replicons referred to herein generally can be plasmids which can replicate in a bacterial host with or without introduced genes. Any introduced genes can include ones that may be expressed in the bacterial host, e.g. $\beta$-galactosidase under control of a bacterial plasmid promoter to allow selection of recombinant plasmids in bacteria, genes which may be expressed only in a eukaryotic host, e.g. $\beta$-galactosidase under control of a eukaryotic (e.g. viral) promoter to allow selection of recombinant viruses in a eukaryotic host, or genes coding for eukaryotic proteins.

One particular example of a plasmid replicon/expression vector system according to the invention is the system based on the polyhedrin gene promoter of the insect *Autographa californica* nuclear polyhedrosis virus (AcNPV), a baculovirus which has been increasingly utilized as an expression vector to synthesize a wide variety of eukaryotic gene products (Smith *et al.*, 1983; Pennock *et al.*, 1984; Miyamoto *et al.*, 1985; Smith *et al.*, 1985; Matsuura *et al.*, 1986, 1987; Possee, 1986; Kuroda *et al.*, 1986; Estes *et al.*, 1987; Inumaru and Roy, 1987, Overton *et al.*, 1987). The basis for expression in this system is the use of the AcNPV polyhedrin promotor, a major late promotor that in wild-type AcNPV infections leads to the production of polyhedrin protein which assembles to form visible polyhedral inclusion bodies that occlude virions in the nucleus of the infected cell.

Replacement of the polyhedrin gene sequence in a suitable plasmid replicon with a foreign gene, followed by transfection of *Spodoptera frugiperda* cells with the derived plasmid in the presence of infectious AcNPV DNA, results in the production of recombinant expression vectors (viruses) that express the foreign gene product *in lieu* of the polyhedrin protein (Smith *et al.*, 1983). Such recombinants can be selected by virtue of their polyhedrin-negative phenotype or other properties, for example those ascribable to the pressure of the foreign gene.

Although the level of expression of the foreign gene product has been found to be variable, recent work has established that the level of expression of a foreign protein in this system is directly related to elements of the DNA sequence between the transcription initiation and the translation initiation site of the polyhedrin gene and that high levels of expression are obtainable when all those sequence elements are present (Matsuura *et al.*, 1987). These studies have resulted in the construction of a new plasmid replicon, pAcYM1, which has been utilized to produce recombinant viruses that express to high levels the gene products of the ambisense S RNA of lymphocytic choriomeningitis (LCMV) virus (Romanowski *et al.*, 1985; *viz*: either the LCMV nucleocapsid protein, N, or the LCMV glycoprotein precursor, GPC). The levels of synthesis of these proteins by the respective recombinant viruses approach those of the polyhedrin protein made in wild-type AcNPV infections (i.e., *ca*. 25-50% of the total cell or insect protein).

Examples of multiple expression vectors according to the invention have been constructed using AcNPV baculovirus expression systems. Using these systems, we have duplicated the AcNPV polyhedrin promoter and its associated transcription termination signals to yield a plasmid replicon, pAcVC2, that contains both the AcNPV polyhedrin gene and the LCMV-N protein each under the control of separately organized polyhedrin promoters. Since both gene products are known to be produced at a high level when expressed individually (Matsuura *et al.*, 1987), the isolation of recombinant viruses using the pAcVC2 plasmid allowed us to assess the ability of the baculovirus system to produce two different gene products simultaneously using the duplicated polyhedrin promoter arrangement. Once the viability of this approach was established, general purpose AcNPV-based plasmid replicons were produced containing a cassette of two PESs with insertion sites for two genes. To minimise recombinational elimination the two PESs were arranged in non-overlapping sequences in opposite orientation (*viz*: separate DNA strands). However the invention does not exclude the possibility of organization in series (*viz*: on the same strand with or without intervening sequences).

General purpose plasmid replicons of the kind described can be used to insert pairs of genes into a viral expression vector. Such pairs or additional pairs of genes can be inserted into the viral genome at multiple sites for polygenic expression by incorporating the corresponding cassettes into intergenic regions (or into non-essential genes) in plasmid replicons containing other parts of the AcNPV genome.

In more detail and by way of example, a copy of the polyhedrin gene promoter of *Autographa californica* nuclear polyhedrosis virus (AcNPV) in association with the coding region of lymphocytic choriomeningitis virus N protein (LCMV-N) and the relevant polyhedrin transcription termination signals, was cloned into the unique *Eco*RV site of a plasmid representing an *Eco* RI derived fragment of the AcNPV genome. The cloning site was upstream of the natural AcNPV polyhedrin gene of the desired recombinant plasmids and the one with the LCMV-N and polyhedrin genes in opposite orientation to each other was selected, to minimise gene elimination by natural recombinational events.

The derived recombinant pAcVC2 plasmid had, therefore, both the normal polyhedrin gene and the LCMV-N gene each with its own copy of the polyhedrin transcriptional machinery.

Co-transfection of *Spodoptera frugiperda* insect cells with the pAcVC2 plasmid together with infectious polyhedrin-negative AcNPV DNA, resulted in the isolation of recombinant viruses that made polyhedrin protein as well as LCMV-N protein. Electron microscopy demonstrated the presence of occluded virus particles in the nucleus of the recombinant virus infected cells and aggregates of LCMV-N protein in the cytoplasm of the same cells. Unlike polyhedra-negative AcNPV recombinants, the occluded recombinants were potent infectious agents for the caterpillar *Trichoplusia ni*.

In a similar manner DNA sequences coding for hepatitis B surface and core antigens have been cloned into unique *Eco*RV and *Acc*11 sites of an *Eco*RI derived fragment of the AcNPV genome. Recombinant plasmids were produced containing (i) the polyhedrin gene and the hepatitis B surface antigen gene and (ii) the hepatitis B surface antigen gene and the hepatitis B core antigen gene.

Co-transfection of *Spodoptera frugiperda* insect cells with these plasmids together with infectious polyhedrin-negative AcNPV DNA, resulted in the isolation of recombinant viruses that made (i) polyhedrin protein as well as hepatitis B surface antigen and (ii) hepatitis B surface antigen and hepatitis B core antigen.

## Examples

The construction of multiple expression vectors according to the invention will be described in more detail in the following Examples.

## EXAMPLE 1

### (i) PROCEDURES

#### A. Construction of the plasmid replicon pAcVC2 containing LCMV-N and AcNPV polyhedrin genes

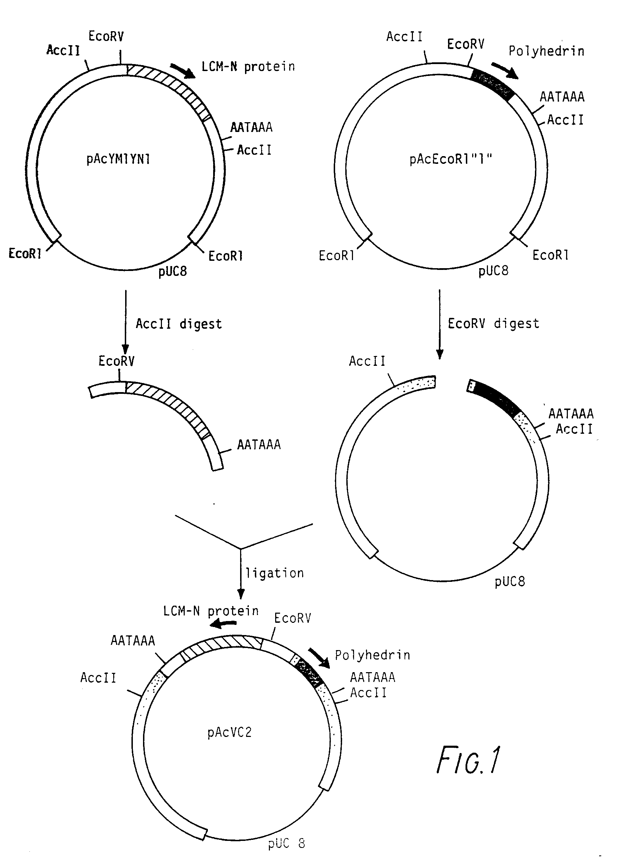

The method chosen to make a baculovirus plasmid replicon that would ultimately contain two gene products under the control of the necessary regulatory sequences required the duplication of the polyhedrin promoter and transcription termination sequences. The transcriptional initiation and terminator sites for the polyhedrin mRNA species have been mapped to, respectively, approximately 48 nucleotides upstream of the translation initiation codon and 376 nucleotides downstream of the translation termination codon (Howard *et al.*, 1986). Since transcription termination occurs downstream of the sequence motif AATAAA, a sequence corresponding to the normal eukaryotic polyadenylation signal (Birnstiel *et al.*, 1985), it has been suggested that baculovirus mRNAs may be cleaved and polyadenylated by the 3'mRNA processing machinery of the hose (see Rohrmann, 1986). By contrast, the precise sequence elements required for the promoter activity of the polyhedrin gene have not been ascertained, although DNA sequence elements commonly observed in association with RNA polymerase II driven transcription can be identified in the sequences that are upstream of the translation initiation site. Therefore, we envisaged that in order to duplicate the required information for the mRNA synthesis and regulation of a foreign gene under the control of the polyhedrin transcriptional machinery a restriction fragment was required that contained DNA sequences some distance upstream of the known transcription initiation site and downstream of the sequences encompassing the replicon termination site. To this end, an available plasmid replicon containing the LCMV-N gene (pAcYM1.YN1, Matsuura *et al.*, 1987) which could yield recombinant viruses that made high levels of LCMV-N protein was used as substrate. It was treated with the restriction endonuclease *Acc*II and the 3052 nucleotide fragment, containing the relevant polyhedrin transcriptional initiation and termination machinery in association with the LCMV-N-gene (Fig. 1), was isolated by standard procedures (Maniatis *et al.*, 1982). Having recovered DNA which should encompass the polyhedrin gene promoter and terminator sequences a plasmid containing the authentic polyhedrin gene was required. The *Eco*RI "I" fragment in pUC8 was selected (Possee, 1986). Initially, the 3052 base pair fragment was placed with the 3′ non-translated region of the polyhedrin gene and in the same orientation as the polyhedrin coding sequence since this region of the AcNPV genome had been utilized previously in our laboratory to insert a synthetic oligonucleotide for the environmental release of a genetically marked baculovirus (Bishop, 1986). However, plasmids containing the *Acc*II fragment in this position did not yield recombinant viruses. Although the reason this did not work is not known, the likely cause was deletion of the inserted gene by homologous sequence recombination. Therefore as an alternative approach, we selected an insertion site in the 5′ upstream region of the polyhedrin gene. Notwithstanding the paucity of biochemical data regarding the sequence elements essential for high level polyhedrin gene promoter activity, the unique *Eco*RV restriction endonuclease site that is located in a non-coding region some 100 nucleotides upstream of the polyhedrin ATG in the AcNPV *Eco*RI "I" fragment was chosen for insertion of the 3052 nucleotide *Acc*II fragment containing the LCMV-N gene. Since the insertion involved a blunt end ligation, the *Acc*II and *Eco*RV sites were destroyed by this procedure. Following ligation and transformation, recombinant plasmids were obtained and analysed by restriction endonuclease digestion. Clones were selected that contained the duplicated promoter and coding region for the LCMV-N gene in the opposite orientation to that of the resident polyhedrin gene (see Fig. 1). Such a configuration should restrict the possibility of homologous sequence genetic recombination and the excision of one or other of the genes of interest from progeny viruses. A derived plasmid replicon, designated pAcVC2, was characterised and shown to have the LCMV-N and polyhedrin genes with their associated polyhedrin transcriptional machinery in the form shown in Figure 1.

B. Preparation of a recombinant baculovirus containing the LCMV-N and the AcNPV polyhedrin genes

The plasmid replicon pAcVC2 was co-transfected into *Spodoptera frugiperda* cells in the presence of an available recombinant, polyhedrin-negative, helper viral DNA. After transfection, putative recombinant viruses (e.g., VC2) were isolated from the infected cells by selecting for progeny virus plaques that exhibited a polyhedrin-positive phenotype (*ca*. 0.1-1% frequency). The helper viral DNA (YM1.BTV-10.2) contained a nucleotide insert representing the major neutralization antigen VP2 of bluetongue virus serotype 10, RNA segment 2 (gift of P. Roy; see Inumaru and Roy, 1987) that had been previously manipulated into the polyhedrin-negative virus using the vector pAcYM1. This helper viral DNA was chosen for two reasons. First, the virus was polyhedrin-negative so that new recombinants could be easily selected from co-transfections involving YM1. BTV-10.2 viral DNA and plasmid replicons containing the polyhedrin gene on the basis of their reacquisition of a polyhedrin-positive phenotype. Secondly, unlike the pAcRP based plasmids, or those derived by Smith and Summers (1983), the helper contained no polyhedrin protein coding sequences (see Matsuura *et al.*, 1987) thereby eliminating the possibility of recombination within the polyhedrin coding region. After three successive cycles of plaque purification stocks of VC2 virus were obtained and used for a range of biochemical analyses.

C. Immunofluorescence analysis of recombinant virus infected cells

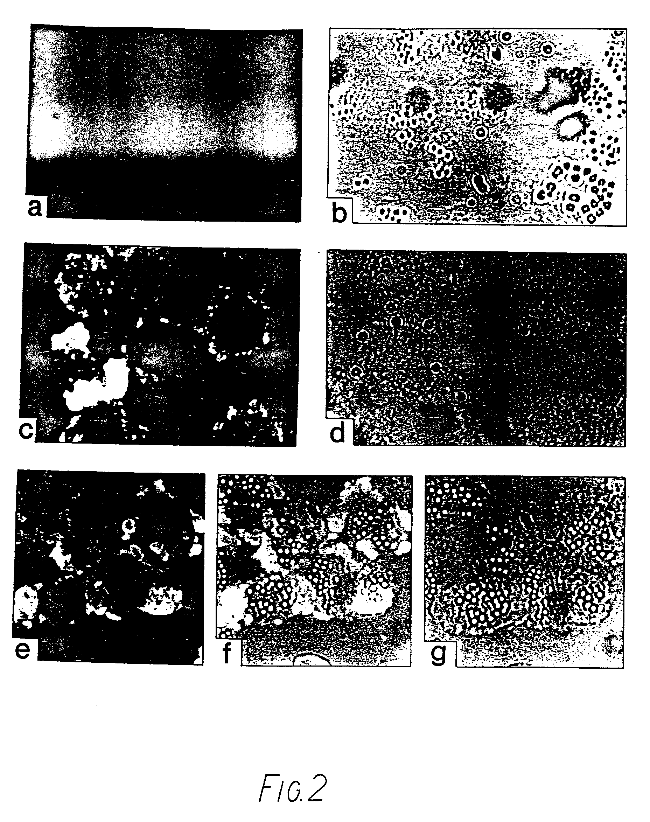

*S.frugiperda* cells were infected with the pAcVC2 derived recombinant VC2 virus and prepared for immunofluorescence analysis using an LCMV-N monoclonal antibody (gift of M.J. Buchmeier, see Buchmeier *et al.*, 1981). As controls, cells infected with either YM1.YN1 virus (containing only the LCMV-N coding region), or wild-type AcNPV were employed. The results, shown in Figure 2, provided evidence for the expression of the LCMV-N protein in the VC2 infected cells (Figure 2e) that was comparable to that observed for the recombinant baculovirus expressing only the LCMV-N protein (YM1.YN1, Figure 2c). As expected, cells infected with wild-type AcNPV did not exhibit positive immunofluorescence (Figure 2a).

In addition to ultraviolet microscopy, examination of virus infected cells using a light microscope revealed the presence of polyhedral inclusion bodies in control wild-type AcNPV infected cells (Figure 2b) and in cells infected with the VC2 recombinant virus (Figure 2g). All the cells infected with the VC2 virus exhibited both LCMV-N immunofluorescence and polyhedral inclusion bodies (compare Figure 2e and 2g). This phenomenon is most clearly seen in the hybrid ultraviolet/visible light micrograph presented in Figure 2f. As shown previously (Matsuura *et al.*, 1987) cells infected with the recombinant virus containing the LCMV-N gene in *lieu* of the polyhedrin gene (i.e., virus YM1.YN1) did not exhibit polyhedral inclusion bodies although they had a marked granular appearance (Figure 2d). In summary, the data clearly demonstrated that cells infected with the VC2 virus expressed LCMV-N protein and polyhedrin simultaneously.

### D. Location of the genes for LCMV-N protein and AcNPV polyhedrin protein within the recombinant VC2 viral DNA

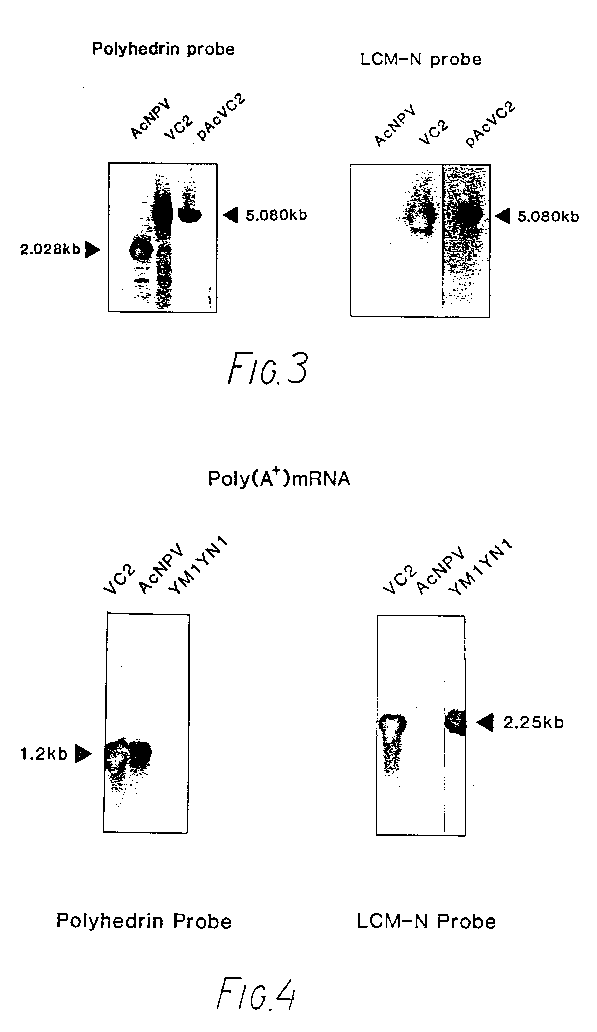

Although the immunofluorescence data shown in Figure 2 indicated that the genes for LCMV-N and polyhedrin were expressed in cells infected with the VC2 virus, it was essential to demonstrate that during the co-transfection regime the genes had been incorporated into the viral genome in the desired orientation (as in pAcVC2, see Figure 1) rather than at random. Conveniently, digestion of the recombinant plasmid pAcVC2 with the restriction endonuclease *Acc*II yields a 5080 nucleotide fragment that contains both the LCMV-N gene and the polyhedrin gene. This enzyme was therefore used for Southern analysis of the recombinant VC2 virus. DNA preparations obtained from pAcVC2 plasmids or from purified VC2, or AcNPV, virions were digested with *Acc*II and resolved by agarose gel electrophoresis. Southern blot analyses of these preparations using radioactive probes specific for the coding region of either the AcNPV polyhedrin or the LCMV-N (Matsuura *et al.*, 1987) gene are shown in Figure 3. The data demonstrated that both the polyhedrin and LCMV-N gene specific probes hybridized to a 5082 nucleotide fragment in the *Acc*II digested VC2 viral DNA that correlated to the *Acc*II fragment detected with both probes in the plasmid replicon pAcVC2. As expected, the polyhedrin probe hybridized to a 2028 nucleotide *Acc*II fragment encompassing the polyhedrin gene in wild-type AcNPV DNA, whilst the LCMV-N probe did not hybridize to any of the AcNPV DNA sequences. The results indicated that both the LCMV and polyhedrin coding sequences were incorporated into the desired area of the VC2 viral genome.

### E. Expression of LCMV-N and polyhedrin gene products by the VC2 virus

The presence of LCMV-N and polyhedrin mRNA species in cells infected with the recombinant VC2 virus was determined by Northern analysis. As controls, *S.frugiperda* cells were infected with AcNPV and the YM1.YN1 recombinant virus (Matsuura *et al.*, 1987). Infected cell nucleic acids were extracted at 24 h post-infection and chromatographed on columns of oligo(dT) cellulose to select RNA with polyadenylic acid sequences. The Poly(A+) and poly (A-) RNA preparations were treated with methylmercuric hydroxide and separated into size classes by agarose gel electrophoresis also in the presence of methylmercuric hydroxide. RNA was blotted and fixed to Hybond-N, then probed with nick-translated DNA representing either the LCMV-N or the AcNPV polyhedrin gene. The results of such analyses are shown in Figure 4. The polyhedrin probe hybridized to a 1.2 kb mRNA species in both the VC2 and AcNPV infected cells but did not hybridize to any mRNA species produced in the YM1.YN1 recombinant virus infection. In contrast, the LCMV-N probe hybridized to an mRNA species *ca*. 2.25 kb in length in both the VC2 and YM1.YN1 virus infected cells. The size of this mRNA species corresponds to the predicted length of the LCMV-N mRNA if the authentic polyhedrin transcriptional machinery has been utilized. These mRNA size analyses confirmed and provided independent evidence that the LCMV-N and polyhedrin genes were under separate transcriptional control. As expected neither polyhedrin, nor LCMV-N, mRNA species were present in the poly(A-) RNA tracts (data not shown). As far as could be ascertained by such analyses, the data exhibited in Figure 4 indicated that the levels of polyhedrin and LCMV-N mRNA in the VC2 virus infected cells were essentially comparable to those produced by cells infected with either AcNPV or YM1.YN1 virus.

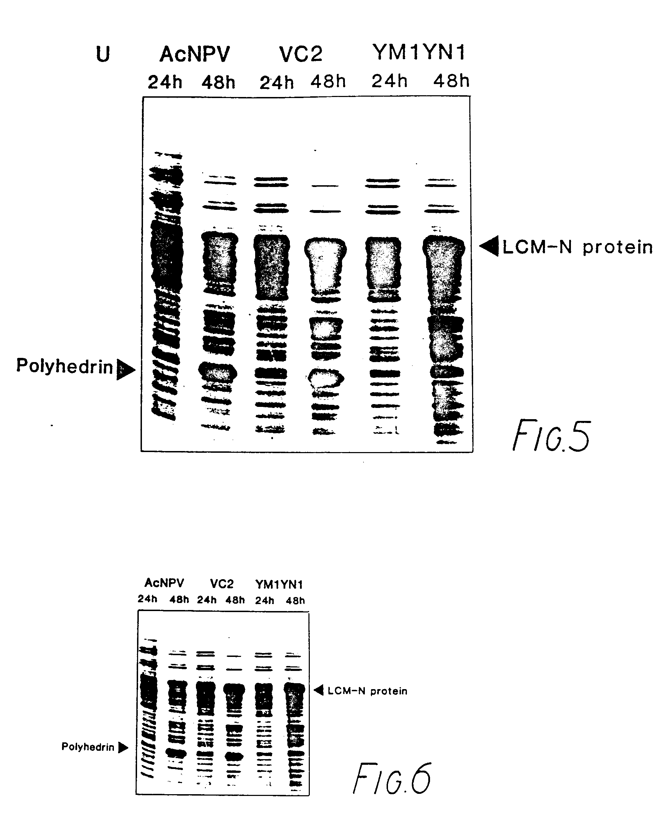

In order to determine the relative amounts of LCMV-N protein and the polyhedrin protein synthesized in the VC2 virus infected *S.frugiperda* cells, cells were pulse-labelled at various times post-infection and the radio-labelled proteins analysed by polyacrylamide gel electrophoresis. The results of pulse-labelling VC2 virus-infected cells with [35S]methionine at 24 h and 48 h post-infection are shown in Figure 5a. It was apparent that in the VC2 virus infected cells there were two major [35S]-labelled protein products with sizes corresponding to 62 x $10^3$ Da and 33 x $10^3$ Da. By comparison with the [35S]-labelled proteins synthesized in insect cells infected with the polyhedrin-negative YM1.YN1 virus, or with the AcNPV virus, the VC2 directed protein products corresponded, respectively, to the LCMV-N protein (62 x $10^3$ Da) and the AcNPV polyhedrin protein (33 x $10^3$ Da). The relative level of LCMV-N and polyhedrin gene expression apparent in the pulse-labelled protein analyses was confirmed by analyses of stained protein gels (Figure 6b). This result also provided evidence that both proteins accumulated to substantial levels in the VC2 virus infected cells.

As discussed above, the LCMV-N gene in the recombinant VC2 virus was located in a position that was upstream, but in the opposite orientation, relative to the normally positioned polyhedrin gene. It is of interest to note, therefore, that the level of LCMV-N expression by the VC2 virus did not differ significantly from that observed with the recombinant baculovirus YM1.YN1 that expresses only LCMV-N (Figures 5 and 6). The level of polyhedrin protein expression in the recombinant VC2 virus infected cells was comparable in molar terms with that obtained for the LCMV-N protein (Figures 5 and 6).

F. Electron microscopic analyses of VC2 virus infected cells

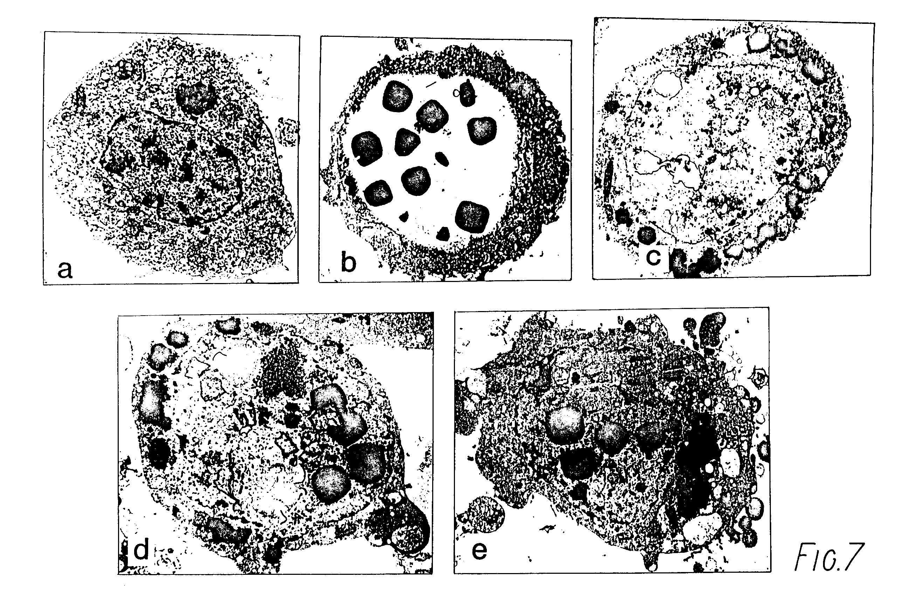

It has been previously noted (Matsuura *et al*., 1987) that electron micrographs of insect cells expressing large amounts of LCMV-N protein contained multiple inclusion bodies in the cytoplasm which, on the basis of the intense cytoplasmic fluorescence observed with LCMV-N monoclonal antibody, were ascribed to LCMV-N protein aggregates. A similar analysis of cells infected with the recombinant VC2 virus is shown in Figure 7. By comparison with uninfected cells (Fig. 7a), AcNPV (Fig. 7b), or YM1.YN1 (Fig. 7c) infected cells, two features of the VC2 virus infections were apparent; firstly, as in the YM1.YN1 virus infected cells, LCMV-N protein was present in aggregated form in cytoplasmic inclusions and secondly, as in wild-type AcNPV infections, the nucleus contained polyhedral bodies (Fig. 7d) with occluded virus particles (Fig. 7e).

G. Expression of LCMV-N protein and polyhedrin in ïT.niô larvae

Since electron micrographs of ïS.frugiperdaô cells infected with the VC2 virus had revealed the presence of VC2 virions occluded with polyhedra, the latter were recovered and used to infect third instar ïT.niô caterpillars. For comparison, cell released virus (titre 8 x $10^7$ pfu/ml) obtained from the supernatant fluids of VC2 virus infected ïS.frugiperdaô cells was also used to infect third instar ïT.niô caterpillars. After four days post-infection and prior to death of the larvae, a random selection of 5 caterpillars from each infection group (40 caterpillars per group) were extracted and their proteins resolved by polyacrylamide gel electrophoresis. The results of the stained protein gel analyses of these caterpillar extracts are shown in Figure 5. Of the five caterpillars that received cell released virus (average caterpillar weight: 97 mg), only caterpillar no. 3 exhibited an infection and the concomitant production of small quantities of LCMV-N protein. In contrast, caterpillars infected with the VC2 derived polyhedral inclusion bodies were significantly smaller in size (average weight: 33 mg) and all five caterpillars exhibited a virulent viral infection with the concomitant production of large quantities of both the LCMV-N and polyhedrin proteins. By comparison with known amounts of bovine serum albumin electrophoresed in parallel with the larval extracts, it was estimated that each larva contained at least 0.1 mg of LCMV-N protein.

(ii) MATERIALS AND METHODS USED IN THIS EXAMPLE

(a) Viruses and cells

AcNPV and recombinant virus stocks were grown and assayed in confluent monolayers of ïSpodoptera frugiperdaô cells in medium containing 10% foetal bovine serum according to the procedures described by Brown and Faulkner (1977).

(b) DNA manipulations and constructions of DNA clones

Plasmid DNA manipulations were effected essentially as summarised by Maniatis and associates (1982). Restriction enzymes, T4 DNA ligase and the Kenow large fragment of DNA polymerase were purchased from Amersham, U.K., Calf intestinal alkaline phosphatase was obtained from Boehringer Mannheim Biochemicals (Mannheim, FRG).

## (c) Construction of pAcVC2

The plasmid replicon pAcYM1.YN1 which contained the entire LCMV-N gene (Matsuura *et al.*, 1987; Possee, 1986) was digested to completion with *Acc*II and the 3052 nucleotide fragment containing the LCMV-N gene with its associated polyhedrin promoter and transcription termination sequences isolated by electrophoresis in 0.8% agarose. The *Acc*II fragment was ligated into an *Eco*RV digested and de-phosphorylated plasmid, pAc*Eco*RI "I", that contained a 7.3 kb AcNPV fragment in a modified pUC8 plasmid (Possee, 1986). After transformation and screening with nick-translated LCMV-N DNA, plasmids were obtained (Figure 1, pAcVC2) that were characterised by restriction enzyme and sequences analysis (Chen and Seeburg, 1985). Those with the format of pAcVC2 shown in Figure 1 were selected to produce the recombinant virus VC2.

## (d) Transfection and selection of recombinant virus VC2

*S.frugiperda* cells were transfected with a mixture of plasmid pAcVC2 DNA and DNA representing a polyhedrin-negative virus derived from pAcYM1 containing the bluetongue virus serotype 10 segment 2 DNA (YM1.BTV-10.2, a gift from Professor P. Roy, University of Alabama in Birmingham, USA, see Inumaru and Roy, 1987) using a modification of the procedures described by Smith *et al.* (1983). YM1.BTV-10.2 DNA (1 $\mu$g) purified by the method of Smith and Summers (1978) was mixed with various concentrations of plasmid DNA (25-100 $\mu$g) and adjusted to 950 $\mu$l with Hepes buffered saline (20 mM Hepes, 1 mM Na$_2$HPO$_4$, 5mM KCl, 140 mM NaCl, 10 mM glucose, pH 7.05). After precipitation with 50 $\mu$l of 2.5 M CaCl$_2$ DNA was inoculated onto monolayers of $1 \times 10^6$ *S.frugiperda* cells in 35 mm tissue culture dishes and incubated for 1 hour at room temperature. The supernates were discarded and 1.5 ml of medium containing 10% foetal bovine serum was added. After 3 days incubation at 28°C, The supernates were harvested and titred in confluent monolayers of *S.frugiperda* cells. Plaques exhibiting occlusion bodies (viral polyhedra, as determined by transmission light microscopy) were recovered and retitred on *S.fruigperda* cells to obtain recombinant, polyhedrin positive virus. Following a third plaque purification, high titred stocks of recombinant virus were obtained (VC2, $10^7$ to $10^8$ pfu/ml).

## (e) Extraction and characterisation of viral and cellular nucleic acids

Viral DNA and infected cell mRNA were prepared as described previously (Matsuura *et al.*, 1986). For Southern analyses, viral DNA was digested to completion with *Acc*II and the products resolved by electrophoresis in 0.8% Agarose (BRL, Madison, W.I.), then blotted to Hybond-N (Amersham, U.K.). After drying, the membranes were illuminated with ultraviolet light for 5 minutes and hybridized (Southern, 1975) to either nick-translated LCMV WE DNA obtained from clone Y-1-A (Matsuura *et al.*,1986), or to nick-translated AcNPV polyhedrin DNA (a gift from Dr. R.D. Possee, Institute of Virology, Oxford, U.K.). The membranes were washed and autoradiographed. Cellular mRNA preparations were treated with 10 mM methyl mercury hydroxide (Bailey and Davidson, 1976), resolved by electrophoresis in 1% agarose gels containing methyl mercury and transferred by blotting to Hybond-N. After blotting (Alwine *et al.*, 1977), the membranes were dried and illuminated with ultraviolet light for 5 min, then hybridized to $^{32}$P-labelled nick-translation products of DNA representing the polyhedrin gene and LCMV-N gene as described by Denhardt (1966). Membranes were washed and autoradiographed.

## (f) Protein analysis

*S.frugiperda* cells were infected with virus at a multiplicity of 10 pfu/cell in 35 mm tissue culture dishes and labelled with 100 $\mu$Ci[$^{35}$S]-methionine (Amersham, 1131 Ci/mmol) for 1 h at the indicated time using methionine-free medium. Prior to labelling, the cells were incubated for 1 h in methionine-free medium to reduce the intracellular pools of the precursor. After the labelling periods, the media was removed, the monolayers rinsed three times with phosphate-buffered saline (PBS) and the cells lysed in 100 $\mu$l RIPA buffer (1% Triton X-100, 1% sodium deoxycholate, 0.5 M NaCl, 0.05 M Tris-HCl, 0.01 M EDTA, 0.1% SDS, pH 7.4). Aliquots of the protein samples were boiled for 5 min in dissociation buffer (2.3% SDS, 10% glycerol, 5% $\beta$-mercaptoethanol, 62.5 mM Tris-HCL, 0.01% bromophenol blue, pH 6.8) and subjected to electrophoresis in discontinuous gels of 10% polyacrylamide as described by Laemmli (1970). After electrophoresis the gels were fixed in acetic acid (10% v/v) and impregnated with Amplify (Amersham, U.K.) and exposed at -70° to X-ray film. Alternatively, gels were stained with Kenacid Blue (Overton *et al.*, 1987).

### (g) Infection of *T.ni* with recombinant VC2 virus

Third instar *T.ni* caterpillars grown on semi-synthetic media (Hoffman *et al.*, 1966) were infected *per os* with either cell-released, non-occluded, VC2 virus purified by differential centrifugation (8 x $10^4$ p.f.u./caterpillar), or VC2 polyhedral inclusion bodies (likewise purified, 4 x $10^4$ polyhedral inclusion bodies per caterpillar). After 4 days of incubation the caterpillars were harvested. The caterpillars were weighed and individuals were selected at random and homogenised in PBS (200 $\mu$l) containing 0.02% sodium diethyl-dithiocarbamate. Aliquots (5 $\mu$l) from the homogenates were prepared for gel electrophoresis.

### (h) Immunofluorescence analysis of recombinant virus infected cells

*S.frugiperda* cells were infected with the VC2 recombinant virus and prepared for immunofluorescence analyses 36 h post-infection using an LCMV-N monoclonal antibody (Buchmeier *et al.*, 1981). As controls, *S.frugiperda* cells infected with either YM1.YN1 virus (Matsuura, *et al.*, 1987; containing only the LCMV-N coding region), or with wild-type AcNPV, were employed. For immunofluorescent analyses, aliquots (5 $\mu$l) of *S.frugiperda* cells infected 36 h previously at a multiplicity of 10 pfu/cell were spotted onto glass slides and fixed with cold acetone for 10 min. The cells were washed in PBS and incubated for 1 h at 37°C with 10 $\mu$l of a 1/32 dilution of LCMV N-specific monoclonal antibody, kindly provided by Dr. M. Buchmeier (Scripps Clinic and Research Institute, La Jolla, Ca., U.S.A.). The slides were washed with PBS, the cells stained with fluorescein conjugated swine anti-mouse IgG antibody for 1 h at 37°C, washed again with PBS and examined for fluorescence.

### (j) Construction of pAcVC3

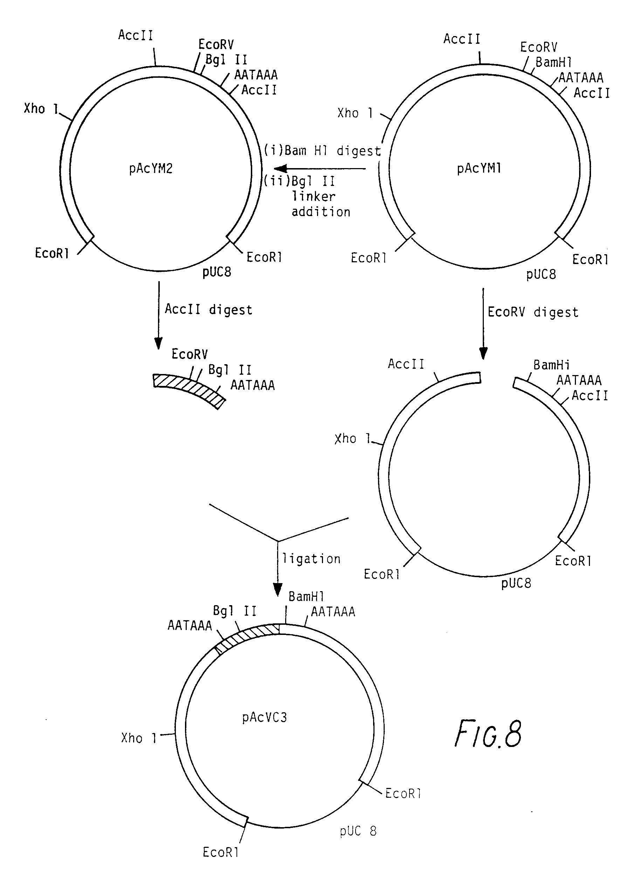

Plasmid pAcYM1 was digested with the restriction enzyme *Bam*HI and the 3$'$ ends repaired with the Klenow fragment of DNA polymerase in the presence of all 4 deoxynucleoside triphosphates. The DNA was then dephosphorylated by treatment with alkaline phosphatase and ligated to a *Bgl*II linker (5$'$ GAAGATCTTC 3$'$). Subsequent digestion with *Bgl*II followed by religation led to the recovery of a new vector, pAcYM2, containing a unique *Bgl*II site in lieu of the original *Bam*HI site (Fig. 8).

To generate a plasmid in which two foreign genes could be inserted, each under the control of its own copy of the polyhedrin promoter, the 1.3 kb DNA fragment obtained by digestion of pAcYM2 with *Acc*II was cloned into plasmid pAcYM1 that had been previously digested with *Eco*RV (Fig. 8). The ligation did not regenerate the *Acc*II or *Eco*RV sites used in these digestions. Of the 2 possible orientations in which the fragment could be recovered in the new recombinant plasmid, the one with the polyhedrin transcription initiation sites next to each other but in opposite orientations was selected, pAcVC3 (Fig. 8). In this orientation the two putative transcription termination signals, AATAAA, were separated by the *Bgl*II and *Bam*HI sites (Fig. 8). The construction of plasmid pAcVC3 allows one foreign gene to be inserted in the plasmid at the *Bam*HI site and another into the *Bgl*II site. By this means each gene has its own, non-overlapping, copy of the polyhedrin transcription promoter and terminator sequences (i.e., representing two PESs). The orientation of the genes on different strands of the DNA, should minimize the possibility of gene excision by recombinational events.

### (k) Preparation of recombinant virus VC4

Using the procedure described above for the production of virus VC2, but employing an available plasmid containing the gene coding for the Hantaan N protein (the protein corresponding to the Hantaan S (small) RNA - Schmaljohn, C.S.; Jennings, G.B.; Hay, J. and Dalrymple, J.M. "Coding stategy of the S-genome segment of Hantaan Virus"; Virology, *155*, pp. 633-643 (1986)), recombinant virus VC4 was produced. This virus, when used to infect *T.ni* caterpillars enabled the concomitant production of polyhedrin protein and Hantaan N protein in those insects.

Using similar techniques, recombinant virus capable of expressing combinations of polyhedrin protein and Hepatitis B, S or C proteins may be produced.

EXAMPLE 2

(i) PROCEDURES

A. Construction of Recombinant Transfer Vectors for Single Gene Expression

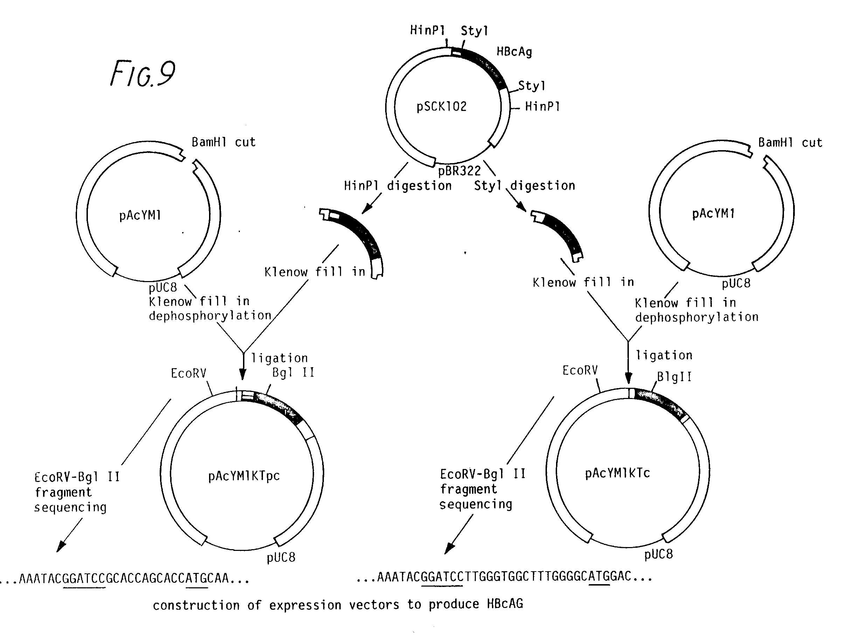

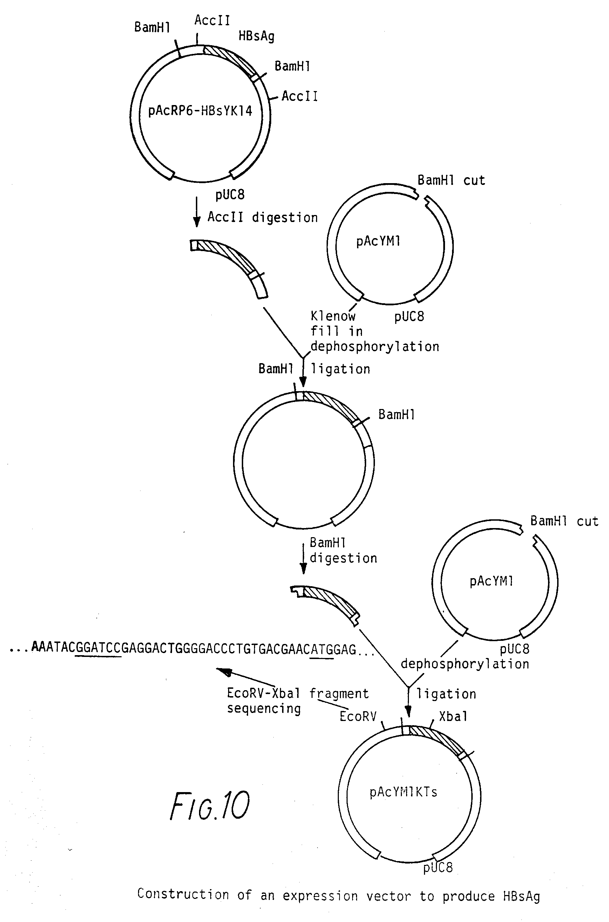

A 581 base-pair DNA fragment containing the coding region of the HB virus C gene and 1005 base-pair fragment containing the coding region of the preC and C gene were excised with *Sty*I and *Hin*PI, respectively, from plasmid pSCK102 kindly supplied by C.-Y. Kang (University of Ottawa, Canada, representing an *adw* serotype of HB virus). Each fragment was repaired with the Klenow fragment of DNA polymerase, then cloned into the *Bam*H1 site of the baculovirus pAcYM1 vector (Matsuura *et al.*, 1987). The derived recombinant transfer vectors were designated pAcYM1KTc and pAcY1KTpc respectively (Fig. 9). The entire S gene of HB virus was prepared by *Acc*II digestion of plasmid pAcRP6-HBsYK14 (Kang *et al.*, 1987), ligated into the *Bam*H1 site of pAcYM1, then digested with *Bam*H1, and the resulting 725 base-pair DNA fragment containing the S gene inserted into the *Bam*H1 site of the pAcYM1 vector to construct the recombinant transfer vector pAcYM1KTs (Fig. 10).

B. Construction of Recombinant Transfer Vectors for Dual Gene Expression

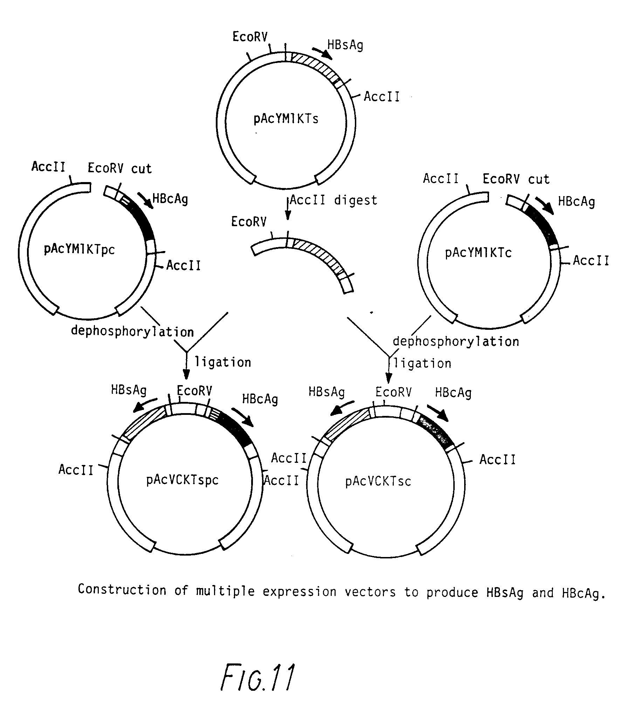

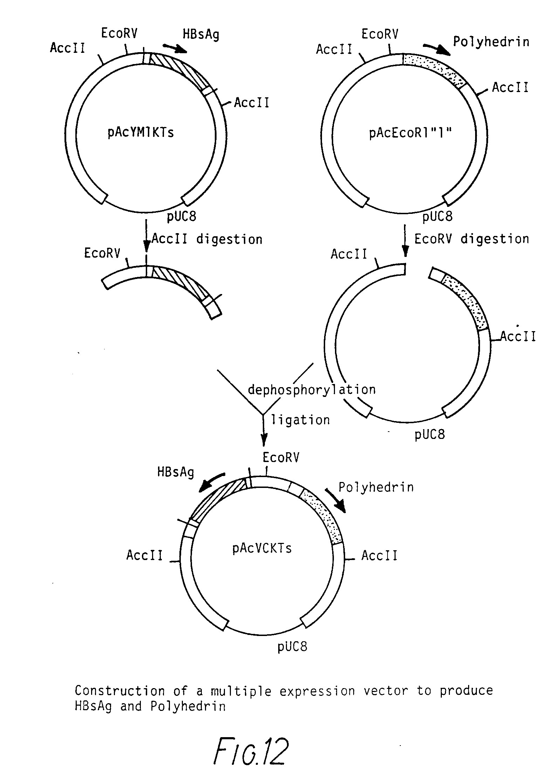

Plasmid pAcYM1KTs was digested with *Acc*II and the fragment containing the S gene, its associated polyhedrin promoter and transcription termination sequences was ligated into the dephosphorylated *Eco*RV digestion product of plasmid pAcYM1KTc to give the dual expression, recombinant transfer vector pAcVCKTsc, (Fig. 11), or into the *Eco*RV digestion product of pAcYM1KTpc to give the dual expression vector pAcVCKTspc, (Fig. 11). Insertion of the same HB S gene fragment into the 7.3 kb *Eco*RI 'I' fragment of AcNPV (in a modified pUC8 plasmid, Possee, 1986) yielded the recombinant transfer vector pAcVCKTs (Fig. 12).

C. Transfections and Selection of Recombinant Viruses

To obtain recombinant viruses that would express the foreign gene(s), *S.frugiperda* cells were transfected with mixtures of infectious AcNPV DNA and plasmid DNA representing the individual recombinant transfer vectors essentially as described by Overton and associates (1987). From cotransfection with pAcYM1KTc plasmid DNA recombinant virus YM1KTc was obtained, likewise from pAcYM1KTpc recombinant YM1KTpc, from pAcYM1KTs recombinant YM1KTs, from pAcVCKTspc recombinant VCKTspc, and from pAcVCKTsc recombinant VCKTsc. To derive an occluded recombinant virus that expressed both the HB S antigen and AcNPV polyhedrin protein, *S.frugiperda* cells were transfected with a mixture of plasmid pAcVCKTs DNA and DNA representing a polyhedrin negative virus derived from pAcSl.10.2 that contained the bluetongue virus serotype 10 segment DNA2 (Immumaru & Roy, 1987). Plaques of recombinant viruses (VCKTs) that contained visible occlusion bodies were recovered.

D. Extraction and Characterisation of Viral DNA

Viral DNA was prepared as described previously (Overton *et al.*, 1987). DNA samples were digested to completion with *Acc*II and the products subjected to Southern analyses as described by Matsuura and associates (1986).

E. Labelling and Analyses of Infected Cell Polypeptides

*S.frugiperda* cells were infected with virus at a multiplicity of 10 p.f.u./cell in 35 mm tissue culture dishes and incubated at 28°C for 48 h. At the indicated times the cells were treated for 1 h with methionine-free medium, then labelled for 3 h with 15 $\mu$Ci of [$^{35}$S]methionine (Amersham, International, 1131 Ci/mmol) in the same medium. On occasion, the cells were not labelled. The cells were rinsed three times with phosphate-buffered saline (PBS) and lysed in 150 $\mu$l of RIPA buffer (1% Triton X-100, 1% sodium deoxycholate, 0.5 M NaCl, 0.5 M Tris-HCl, 0.01 M-EDTA, 0.1% SDS, pH 7.4). Portions of the protein samples were boiled for 5 min in dissociation buffer (2.3% SDS, 10% glycerol, 5% $\beta$-mercaptoethanol, 62.5 mM-Tris-HCl, 0.01% bromophenol blue, pH 6.8) and subjected to electrophoresis (SDS-PAGE) in a discontinuous gel of 10-20% polyacrylamide containing SDS as described by Laemmli (1970). After electrophoresis the gels were fixed in acetic acid (10% v/v) and stained with Kenacid Blue, then exposed at -70°C to X-ray film.

F. Immunoblotting Analysis

After SDS-PAGE, proteins were transferred electrophoretically to nitrocellulose membranes for 4 h at 150 mA. The membranes were soaked at 4°C overnight in TBS (20 mM-Tris-HCl, pH7.4, 0.15 M-NaCl containing 10% foetal calf serum). After another wash with TBS, the membranes were treated for 1 h at 20°C with rabbit anti-HBcAg serum in TBS containing 5% foetal calf serum and 0.05% Tween 20 (TTBS). Following further washes with TTBS, bound antibodies were detected with goat antirabbit immunoglobulins conjugated to alkaline phosphatase (Sigma) and Fast Blue BB salt and $\beta$-naphthylphosphate (Sigma) as substrate.

G. Purification of HBcAg and HBsAg by Gradient Centrifugation

Cells were infected with recombinant baculoviruses containing the HBsAg gene (YM1KTs) and the supernatant fluids recovered 4 or 5 days post-infection. The cell culture supernatant fluids containing HBsAg were concentrated by precipitation with 60% ammonium sulphate. The pelleted material was resuspended in TNE (10 mM-Tris-HCl, 50 mM-NaCl, 0.1 mM-EDTA, pH 7.4) and loaded on a 20% to 60% (wt/wt) sucrose gradient in TNE buffer and centrifuged at 150,000 x g for 15 h at 4°C using an SW 41 Ti rotor (Beckman). After centrifugation, the gradients were fractionated and peak fractions containing HBsAg identified by RIA (see above), pooled and pelleted by centrifugation (Ti 50 rotor, 100,000 x g for 15 h at 4°C). The products were resuspended in $H_2O$, and samples assayed by electron microscopy. Cells infected with recombinant viruses containing HBcAg were extracted 4 days post-infection by sonication, or freezethawing three times, the products subjected to centrifugation to remove cell debris and the derived supernatant fluids centrifuged through a 30% sucrose cushion using a 42.1 rotor (Beckman) at 106,000 x g for 16 h, then resuspended in TNE and purified by CsCl isopycnic centrifugation using an SW41 rotor at 160,000xg for 36 h. The gradient was fractionated and the peak fractions containing NBcAg were identified by SDS-PAGE, pooled, pelleted by centrifugation and resuspended in $H_2O$.

H. Infection of *Trichoplusia ni* with Recombinant Viruses that Express HBsAg

Groups of 10-20 fourth instar *T.ni* caterpillars grown on semi-synthetic medium (Hoffman *et al.*, 1966) were infected *per os* with non-occluded recombinant virus YM1KTs (5 x $10^5$ p.f.a./lava) or by polyhedral inclusion bodies (PIBS) representing the recombinant VCKTs virus (4 x $10^4$ PIBS/caterpillar). After 4-5 days of extrinsic incubation the visibly infected and moribund caterpillars were harvested, homogenized in 200 $\mu$l of 10 mM-Tris-HCl, pH 7.4 containing 0.02% sodium diethyldithiocarbamate. Portions (5 $\mu$l) of the homogenates were prepared for SDS-PAGE.

I. RIA for Detection and Quantitation of HBsAG

Recombinant virus derived HBsAg was measured by solid-phase radioimmune assay (AUSTRIA II; Abbott Laboratories) using the HBsAg supplied by the manufacturer (20 mg/ml) as a standard.

J. Serology

Immunofluorescence was used to detect recombinant virus-derived HBsAg and HBcAg in infected cells.
Antibodies to HBsAg (anti-HBs) in human sera were measured using a commercially available RIA (AUSAB; Abbott Laboratories) and by two solid-phase "sandwich" ELISA procedures. ELISA No. 1 used human plasma-derived HBsAg for antibody capture and detection. ELISA No. 2 used recombinant virus-derived HBsAg for antibody capture and human plasma-derived HBsAg for antibody detection. Antibodies to HBcAg (anti-HBc) were measured using two solid-phase competitive RIA.RIA No. 1 used human liver-derived HBsAg; RIA No. 2 used recombinant virus-derived HBcAg.

K. Immunofluorescence Analyses

*S.frugiperda* cells were infected with virus at a multiplicity of 1 p.fu./cell in 35 mm tissue culture dishes. At 72 h post-infection portions of 5 $\mu$l were spotted onto PTFE-coated multispot microscope slides and fixed with cold acetone for 10 min. Uninfected (control) cells were treated similarly. Sera and ascitic fluids were diluted 10-fold with PBS. 10 $\mu$l volumes of each preparation were pipetted on to one area of infected cells and one area of control cells. Slides were incubated for 30 min at 37°C in a sealed box at high humidity. Excess antibody was rinsed from the slides with PBS, the slides were drained and any remaining liquid was removed. Appropriate conjugates were diluted in PBS containing 0.005% Evans Blue to mask non-specific (background) fluorescence. Sheep anti-human immunoglobulin-fluorescein isothiocyanate (FITC) (Wellcome Diagnostics, Dartford, UK) and goat anti-mouse IgG and IgM-FITC (TAGO, Inc., Burlingame, California, USA) were diluted to provide bright fluorescence with positive sera and low background staining with negative sera and on control cells. 10 $\mu$l volumes of diluted conjugate were pipetted on to each area of cells and the slides incubated at 37°C for 30 min in a sealed box that provided a humid environment. The stained slides were rinsed and washed as before and finally rinsed for 1 min in distilled water. After drying in air the slides were examined using a fluorescence microscope.

### L. RIA to Measure Anti-HBs

Antibodies to HBsAg were measured in a commercially available RIA (AUSAB) by comparison with the WHO reference anti-HBs preparation which was diluted in negative human serum to contain 100,50 and 10 International Units (IU)/L.

### M. ELISA to Measure Anti-HBs

The solid-phase "sandwich" ELISA used to measure anti-HBs included a novel enhancement system developed in the Division of Microbiological Reagents and Quality Control (CPHL, Colindale). FITC-conjugated antigens or antibodies are detected by a peroxidase conjugate of the monoclonal anti-FITC antibody described by Samuel and associates (1988). In this anti-HBs assay, test antibody was sandwiched between HBsAg-coated microwells and an HBsAg-FITC conjugate. Human plasma-derived HBsAg was conjugated to FITC as described by Samuel and associates (1988) using 1 mg HBsAg and 37.5 $\mu$g FITC in 0.5 ml of 0.1 M-sodium carbonate buffer, pH 9.3, containing 0.1 M-NaCl. An anti-FITC peroxidase conjugate prepared by the periodate method of Wilson and Nasane (1978) was used to detect bound antigen-FITC.

Human plasma-derived HBsAg was purified from human serum and recombinant virus derived HBsAg was purified from cell culture supernatant fluids by affinity chromatography using a column of Protein-A Sepharose CL4B (Pharmacia, Uppsala, Sweden) to which monoclonal anti-HBs has been cross-linked using dimethylsuberimidate (Davies & Stark, 1970; Parkhouse, 1984). The antigen was recovered and diluted in 0.1-M-glycine-HC1 buffer, pH 7.5, containing 0.1% sodium azide, to give a concentration of 1 $\mu$g/ml and used to coat polystyrene microtiter plates (Nunc-Immuno plate Maxisorp F96, A/S Nunc. Kampstrup, Denmark). 100 $\mu$l of antigen suspension were pipetted into each well and the plates incubated at room temperature for three days. They were then washed and unused binding sites blocked with 1% BSA in 0.02-M-Tris-HC1 buffer pH 7.6, containing 0.1% sodium azide. Plates were stored at 4°C until required. 100 $\mu$l of patient serum, either from HBsAg vaccinees, or from patients with a history of hepatitis B infection, as well as appropriate positive and negative controls, were added to the HBsAg coated wells and incubated at 37°C for 1 h. The wells were washed to remove unbound material and 100 $\mu$l of human plasma-derived HBsAg-FITC conjugated buffer (PBS containing 0.05% Tween 20,5% negative human serum and 1% BSA) were added. Following incubation for 1 h at 37°C and washing of the wells, 100 $\mu$l of monoclonal anti-FITC-peroxidase conjugate, diluted in conjugate buffer, was allowed to react with the bound HBsAg-FITC. After incubation for 1 h at 37°C and a final washing step, a substrate solution containing hydrogen peroxide and the chromogen tetramethylbenzidine (TMB) was added (0.001 g TMB [dissolved in 100 $\mu$l diethylsulphoxide] and 7.5 $\mu$l of 6% w/v hydrogen perioxide in 10 ml 0.1-M-citrate-acetate buffer, pH 6.0). The enzyme reaction was stopped after 30 min by the addition of 100 $\mu$l of 2-M-$H_2SO_4$. The absorbances of the controls and the test samples were measured at 450 nm.

### N. RIA to Measure Anti-HBc

The solid-phase competitive RIA used were modifications of the method described by Cohen and associates (1981). Human liver-derived HBcAg was diluted $1.4 \times 10^3$-fold and recombinant virus-derived HBcAg was diluted $2 \times 10^5$-fold in 0.02-M-Tris-HC1 buffer, pH 7.6, containing 0.1% sodium azide. Two hundred and ten etched polystyrene beads (Northumbria Biologicals, Cramlington, UK) were added to 35 ml of each diluted antigen in small flasks which were shaken at room temperature for 2 h, then incubated at room temperature in the dark for a further 2-3 days. The HBcAg-beads were washed three times in PBS and stored until required at 4°C in PBS containing 0.5% BSA and 0.1% sodium azide. 20 $\mu$l of the test

sera were aded to each well of an assay plate followed by 180 $\mu$l of $^{125}$I-labelled anti-HBc diluted in PBS containing 10% FCS. A negative serum, a strong positive and a weak positive serum served as controls and were similarly processed. Excess buffer was removed from the HBcAg-beads by blotting and one bead was added to each well. The wells were sealed and the plates incubated overnight in a humid atmosphere at room temperature. The wells were washed with distilled water using a Qwikwash (Abbot Laboratories) and then the beads were transferred to counting tubes and counted for radioactivity in a 16-well gamma-counter (NE 1600, Nuclear Enterprises, Edinburgh, Scotland, UK). Background (Bkgd) radiation was also measured. The % inhibition of the $^{125}$I-antiHBc binding was expressed as:

100-[{count-bkgd}x100 + {mean negative count-bkgd}]

In these assays, "strong" positive control sera gave 94% inhibition and "weak" positive control sera gave 76% inhibition. Similar values were obtained for both the recombinant virus-derived and the liver-derived antigen. The positive cut-off value was 67% inhibition and the negative cut-off value was 50% inhibition.

(ii) <u>RESULTS</u>

A. <u>Sequence Analyses of HB Virus C and PreC Genes</u>

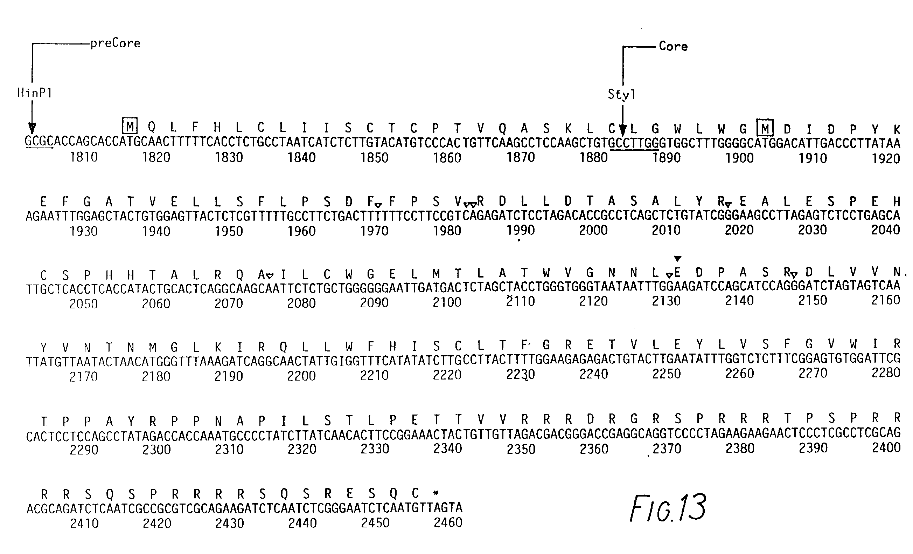

DNA subclones encompassing the C and preC antigens of an adw serotype of HB virus were obtained as described above. The sequences of the HBcAg and HBpcAg gene products were deduced from DNA analyses of these clones (Fig. 13). Indicated in the Figure are the methionine codons (boxed) that initiate the preC and C gene products, as well as nucleotide (open triangles) and amino acid differences (filled triangles) by comparison with the HB virus *adw* sequence published by Ono and associates (1983). The sequences of the indicated gene products were comparable to those published for other isolates of HB virus (see Galibert *et al.*, 1979; Pasek *et al.*, 1979; Velanzuela *et al.*, 1980; Fujiyama *et al.*, 1983; Ono *et al.*, 1983; Bichko *et al.*, 1985).

B. <u>Recombinant Baculoviruses That Express HBcAg, or HBpcAg, or HBsAg</u>

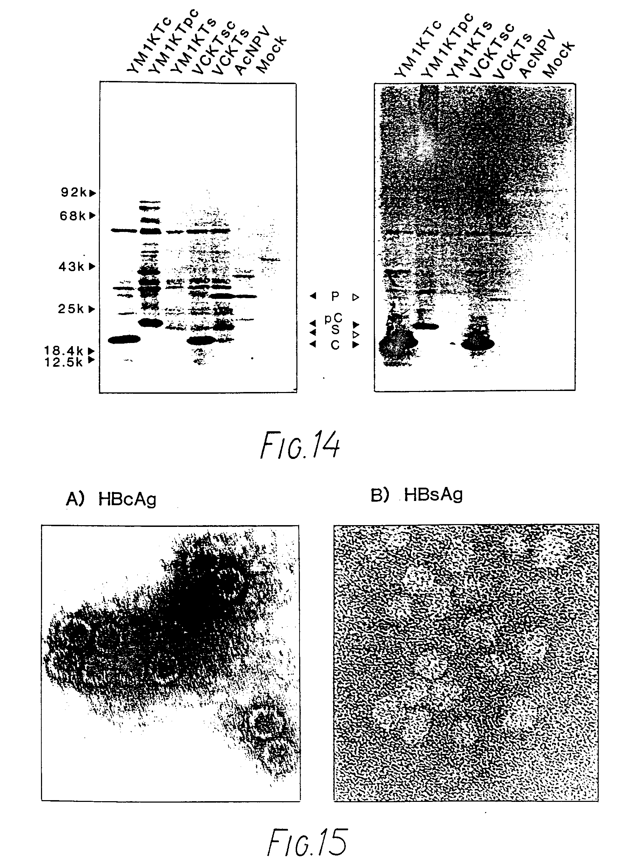

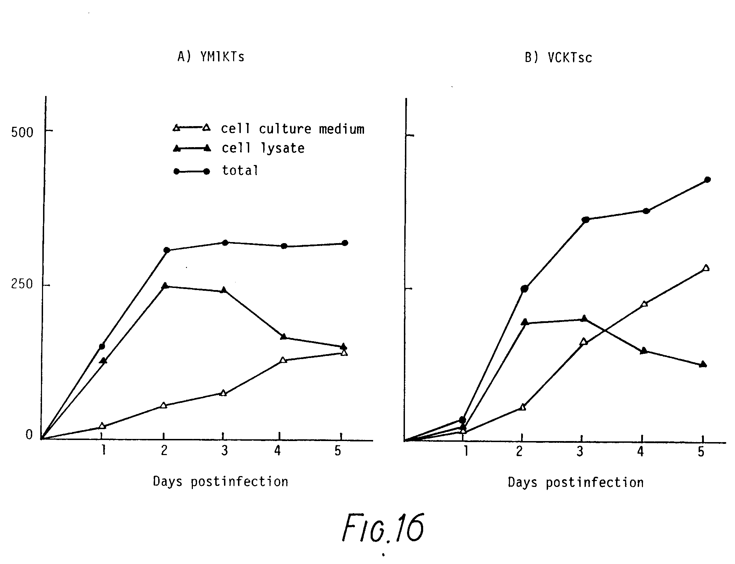

The coding sequences of the preC and C antigens of HB virus were inserted into the AcNPV transfer vector pAcYM1 (Matsuura *et al.*, 1987) as described above (Fig. 9). The derived recombinant transfer vectors (pAcYM1KTpc, pAcYM1KTc) were analysed by restriction endonuclease digestion and the junction sequences determined as shown in Fig. 9. Following cotransfection with infectious AcNPV DNA, recombinant baculoviruses were obtained (YM1KTpc, YM1KTc), plaque purified and high titred virus stocks obtained. The levels and identities of the 24.6 kilodalton HBpcAg and 21.4 kilodalton HBcAg were determined by the incorporated of labelled methionine (Fig. 14, left-hand panel) and by Western analyses (Fig. 14 right-hand panel). Essentially all the HBcAg and HBpcAg antigen was cell associated. Electron micrographs of HBcAg extracted from infected cells and purified by CsCl gradient centrifugation are shown in Fig. 15A. From analyses of stained protein gels of the gradient purified HBcAg, by comparison with known amounts of bovine serum albumin, the yield of <u>purified</u> HBcAg was determined to be of the order of 5 mg per liter of 1 x $10^9$ infected cells.

Using small volume cell cultures (e.g. 35 mm dishes), the level of HBsAg synthesis by the recombinant baculovirus HBsYK14 (Kang *et al.*, 1987) has been reported to be equivalent to 1.5 mg per $10^9$ infected cells. In large culture volumes (100-1000 ml), expression levels of the order of 0.3-0.5 mg per $10^9$ infected cells are obtained with the same virus. The reason for this difference in expression level is not known. In order to improve this the HBS gene was transferred from the pAcRP6 vector to the pAcYM1 vector since higher expression has been observed with the latter for a variety of gene products (see Matsuura *et al.*, 1987). In an initial study, the HBsAg gene was recovered from the transfer vector pAcRP6-HBsYK14 (Kang *et al.*, 1987) by *Bam*HI digestion and inserted directly into the *Bam*HI site of transfer vector pAcYM1. The derived transfer vector was used to prepare a recombinant virus and the level of HBsAg synthesis was measured. The data obtained (not shown) indicated that the level of expression was equivalent to 0.3 mg per $10^9$ infected cells and similar to that of the recombinant HBsYK14 run in parallel.

One reason for the low expression level could have been the presence of some 100 nucleotides of the HB genome upstream of the HBsAg coding region (i.e., between the HB *Bam*HI site and the HBsAg translation initiation ATG). In view of this, the transfer vector pAcYM1KTs was prepared (Fig. 10). This vector was used to produce a recombinant virus (YM1KTs). The presence of HBsAg in the supernatant fluids (containing the secreted 22 nm HBsAg particles) and residual HBsAg in cell lysates was measured. The yield of HBsAg (cell associated and in the supernatant fluids) was estimated from the RIA assays to be of the order of 0.3 mg per liter of $1 \times 10^9$ infected cells by 5 days post-infection (Fig. 16). This level of HBsAg expression was no different to that obtained with the other recombinants (e.g. HBsYK14). The incorporation of labelled methionine into the cell-associated HBsAg was also measured (Fig. 14, see Kang *et al.*, 1987). Electron micrographs of HBsAg purified from the supernatant fluids by sucrose gradient centrifugation are shown in Fig. 15B.

## C. Recombinant Baculoviruses That Coexpress HBcAg and HBsAg

A sequence containing the polyhedrin promoter, the HBsAg gene and the polyhedrin transcription termination signal was recovered from the HBsAg transfer vector pAcYM1KTs by *Acc*II digestion (Fig. 11). It was inserted into the *Eco*RV sites of the transfer vectors pAcYM1KTpc and pAcYM1KTc in order to make dual recombinant vectors (Fig. 3). The recombinant vectors were characterised and the vector with the HBsAg gene in the opposite orientation to the C gene (pAcVCKTspc see Fig. 11) were used to derive recombinant viruses by cotransfection of *S.frugiperda* cells in the presence of infectious AcNPV DNA. The recombinant virus VCKTsc were recovered, plaque purified and grown into high titred virus stock. The antigens made by the VCKTsc recombinant were analysed. The synthesis of HBcAg by the VCKTsc recombinant was demonstrated by the incorporation of labelled methionine and by Western analyses (Fig. 14). The level of synthesis of HBcAg was essentially similar to that obtained with the recombinant YM1KTc.

The synthesis and secretion of HBsAg by cells infected with the dual recombinant VCKTsc and the residual cell-associated HBsAg were determined by ELISA assays (Fig. 14). Based on the RIA assays, the total yield of HBsAg was estimated to be of the order of 0.4 mg per liter of $1 \times 10^9$ infected cells by 5 days post-infection (Fig. 16), slightly higher than that obtained with the single expression recombinant virus, YM1KTs, analysed in parallel. The level of secreted HBsAg (Fig. 16) was almost twice that obtained with the recombinant YM1KTs, although the cell associated HBsAg was essentially the same. Overall, though, the level of HBsAg synthesis represented only a small fraction of the amount of HBcAg synthesis.

## D. Recombinant Baculoviruses That Coexpress HBsAg and AcNPV Polyhedrin Protein

The sequence containing the polyhedrin promoter, the HBsAg gene and the polyhedrin transcription termination signal was recovered from the HBsAg transfer vector pAcYM1KTs by *Acc*II digestion (Fig. 12). It was inserted into the EcoRV site of the EcoRI"I" fragment in order to make a dual recombinant vector that contained the HBsAg gene in addition to the AcNPV polyhedrin gene. The recombinant vectors were characterised and those with the HBsAg gene in the opposite orientation to the polyhedrin gene (pAcVCKTs Fig. 12) were used to derive recombinant viruses by cotransfection of *S.frugiperda* cells in the presence of infectious AcNPV DNA. The proteins made in infected *S.frugiperda* cells by recombinant VCKTs are shown in Fig. 14 by comparison with an AcNPV infection so that the AcNPV polyhedrin protein could be identified. Although for the latter only small amounts of polyhedrin were made (m.o.i. that was used was <1 p.f.u./cell), the data served to identify the polyhedrin protein in the VCKTs infected cells. As in the other recombinants that contained the HB S gene, the synthesis of HBsAg was a minor component of the labelled proteins. From RIA it was estimated to represent some 0.4 mg per liter of $1 \times 10^9$ infected cells by 5 days post infection.

## E. Levels of Synthesis of HB Proteins in Recombinant Virus Infected *S.frugiperda* cells

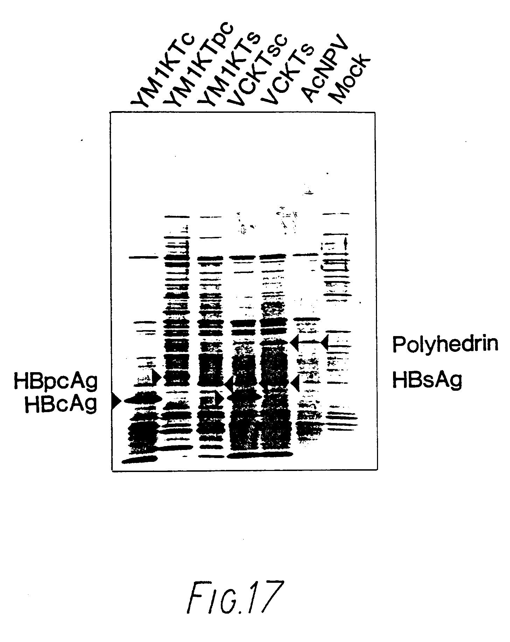

In Fig. 17 are shown the profiles of stained proteins recovered from cells infected with the single expression recombinant viruses YM1KTc, YM1KTpc, and YM1KTs, or the dual expression recombinants, VCKTsc and VCKTs, by comparison with an AcNPV infection and mock virus infected cells. The AcNPV and VCKTs infections were initiated at multiplicities of *ca* p.f.u./cell (accounting for the low levels of polyhedrin protein), the other viruses at 10 p.f.u./cell, and the proteins recovered 2 days post-infection. It was evident that for viruses containing the HB C gene, the HBcAg was present in the cell extracts in substantial quantities (estimated visually to be *ca* 40% of the total protein in the cell extracts). The preC antigen was observed in lower amount (estimated *ca* 5-10% of the stained proteins). Between multiplicities of 1-10 p.f.u./cell essentially similar results to those shown in Fig. 17 were obtained for recombinants containing either the HB C, or the HBpC gene.

Despite the fact that the HBsAg was located in a position in the gel where a cellular protein migrated (see Fig. 17, Mock track), it could be visualized as an enhanced band in the stained gels, although in an amount considerably lower than the amount of HBcAg. Between multiplicities of 1-10 p.f.u./cell essentially similar levels of cell-associated HBsAg were observed by 2 days post-infection, although for the VCKTs virus there were enhanced levels of polyhedrin protein at the higher multiplicities (estimated to be *ca* 40% of the cellular protein, see Fig. 14). Even at the higher multiplicities, it was estimated that the cell-associated HBsAg represented no more than *ca* 2% of the stained proteins.

F. Immunofluorescence Using Recombinant Virus Infected Cells Expressing HBcAg and HBsAg to Identify Human Antibodies to Those Antigens

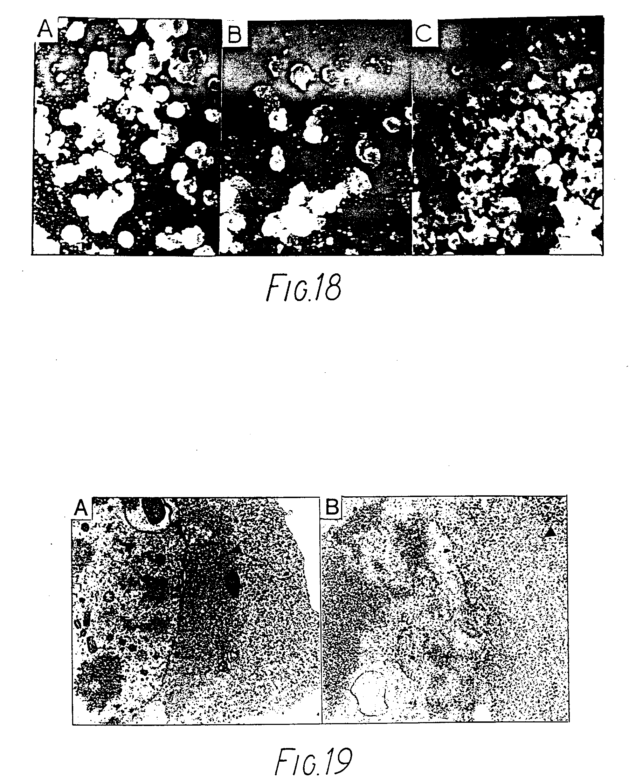

Human sera were provided by the Virus Reference Laboratory of the CPHL, Colindale. Eight human sera that were strongly positive for antibody to HBcAg and negative for antibody to HBsAg in radioimmunoassays were used to stain the slides. Every serum gave good fluorescence on the recombinant VCKTcs virus infected insect cells (Fig. 18, left-hand panel), but not reaction to control, uninfected cells (Fig. 18, right-hand panel), indicating that by this procedure the antigens in the infected cells were suitable substrates for the detection of human antibodies to HBcAg.

Human vaccinee sera with high levels of antibody to HBsAg (up to 500 International Units [IU] per liter), but which contained no anti-HBcAg as judged by the appropriate RIA, gave no fluorescence on the same recombinant virus infected cells. The presence of HBsAg in these virus infected cells was confirmed, however, using a mouse monoclonal anti-HBsAg, giving good fluorescence (Fig. 18, center panel). It was concluded that the cell-associated HBsAg in the virus infected cells was not adequate for identifying such levels of human antibodies to HBsAg.

G. ELISA Assays Using Purified Recombinant Virus Derived HBsAg to Identify Human Antibodies to HBsAg

Eighty-eight sera sent to the Virus Reference Laboratory for anti-HBsAg determination and ninety-one sera from HBsAg-negative blood donations (North London Blood Transfusion Center, Edgware, UK) were tested by RIA and ELISA. The RIA used a non-recombinant HBsAg from Abbott Laboratories. The ELISA No. 1 assay used the human plasma derived HBsAg for both antibody capture and detection. The ELISA No. 2 assay used the recombinant HBsAg purified as described in Methods for antibody capture and a human plasma derived HBsAg for detection. The results were compared to positive control sera prepared by dilution in normal human serum of World Health Organisation (WHO) anti-HBsAg reference serum (RIA), or Blood Products Laboratory (BPL, Elstree, UK) anti-HBsAg reference serum (ELISA). The control sera contained 10,50 and 100 IU/liter of anti-HBsAg antibody. There was good agreement between the tests using the two different antigen sources for antibody capture as shown in Table 1.

H. Competitive RIA Using Purified Recombinant Virus Derived HBcAg to Identify Human Antibodies to HBcAg

One hundred and fifty sera sent to the Virus Reference Laboratory (Colindale) for anti-HBcAg determination were tested by competitive RIA using either the recombinant virus derived HBcAg, or human liver derived HBcAg.

Six of the sera tested gave "weak positive" reactions with one test but "negative" reactions with the other (see above and Table 2). Two sera gave positive reaction in the RIA using liver derived material and a negative reaction using the recombinant antigen. The competitive RIA with human liver-derived HBcAg is known to give some false positive results - possibly due to the serum reacting against liver proteins. In view of this the agreement between the two tests was considered excellent, however, many more sera will have to be tested in the RIA (or by ELISA) with the recombinant virus derived HBcAg to determine the suitability and specificity of such tests.

I. Expression of HBsAg in *T.ni* larvae

When the single expression vector for HBsAg (recombinant YM1KTs) was used to infect fourth instar larvae of *T.ni* (1-2 x $10^5$ p.f.u./larva) it was found that the quantity of HBsAg in moribund larvae was of the order of 1-2 $\mu$g per larva. However at this late larval development stage only 30% of the larvae became infected. Reproducible infection was obtained using earlier instar larvae, however the yields of antigen were correspondingly lower (<0.1 $\mu$g per larva). The use of higher titers of virus to circumvent the problem with fourth instar larvae was not considered practicable without concentrating the virus. Stock virus titers were usually of the order of $10^{7-7.7}$ p.f.u./ml tissue culture fluids.

It has been demonstrated that occluded AcNPV are more effective and reproducible at establishing infections in *T.ni* larvae than non-occluded AcNPV (See Example 1). Individual fourth instar *T.ni* larvae were therefore infected with $4 \times 10^4$ PIBS representing the occluded recombinant VCKTs (see above). All the larvae became infected. The levels of HBsAg obtained in these larvae were of the order of 2.-4 $\mu$g per larva.

J. Electron Microscope Analyses of Cells Infected with Recombinants that Express the HBcAg

In view of the amounts of HBcAg synthesized in *S. frugiperda* cells infected with the recombinant virus YM1KTc, an examination of electron micrographs of cells infected with the virus was undertaken (Fig. 19). Numerous spherical particles were evident in the cells, primarily in the vicinity of the cell nucleus. The sizes of the particles were comparable to those of gradient purified HBcAg (See Fig. 15).

SUMMARY

In order to demonstrate the feasibility of constructing multiple expression vectors that can make large quantities of different foreign proteins in the same cell, we have prepared a baculovirus plasmid replicon, pAcVC2, that contains the complete coding regions of both the AcNPV polyhedrin gene and the LCMV-N gene. Each gene was constructed so that it had its own copy of the AcNPV polyhedrin promoter and transcription termination sequences, although the LCMV-N gene was positioned in an opposite orientation to that of the polyhedrin. The derived plasmid was co-transfected with a polyhedrin-negative viral DNA into *S.frugiperda* cells and recombinant viruses with a polyhedrin-positive phenotype were isolated. Upon analysis by a variety of biochemical procedures it was shown that recombinant viruses, such as VC2, produced both the LCMV-N protein and polyhedrin protein simultaneously and in a regulated fashion (i.e., as major late proteins). The two gene products represented a substantial proportion of the total cellular protein. Such results are significant since they demonstrate that a recombinant baculovirus derived from a plasmid replicon containing a duplicated polyhedrin promoter as illustrated in Figure 1 (e.g., pAcVC2) is capable of making two gene products in large quantities. By constructing a new vector in which the pAcVC2 polyhedrin gene is replaced by an alternative foreign gene and cotransfecting cells with the derived plasmid in the presence of VC2 DNA it should be possible to obtain recombinant polyhedrin-negative viruses that express both foreign proteins simultaneously. Since the level of expression by the polyhedrin transcriptional machinery is related to the representation of the sequences upstream of the polyhedrin ATG (Matsuura *et al.*, 1987), it will be possible to regulate the level of the different foreign genes that are expressed by manipulation of those sequences.

As far as could be ascertained by the experiments that were undertaken, (Northern analyses of the LCMV-N and polyhedrin gene mRNA levels in VC2 virus infected cells) there was not a significant difference between the mRNA levels or expression in molar terms of the two genes nor, as far as could be ascertained from parallel analyses, was there a significant difference in the level of polyhedrin mRNA between cells infected with the VC2 virus by comparison with AcNPV.

Electron micrographs of VC2 virus infected *S.frugiperda* cells have shown that intracellularly polyhedrin assembles to form nuclear inclusion bodies indistinguishable from those found in wild-type AcNPV infections and that the LCMV-N protein aggregates to form cytoplasmic inclusion bodies. Furthermore, VC2 virions are occluded within the polyhedral inclusion bodies. The use of insects (specifically the caterpillar *T.ni*) that are susceptible to baculovirus infection to produce recombinant viral proteins has been demonstrated by various investigators (Maeda *et al.*, 1985; Overton *et al.*, 1987). *In natura*, infection of susceptible caterpillars proceeds by dissolution of the polyhedral inclusion body in the alkaline pH of the insect midgut to liberate virions which then infect the susceptible midgut cells. In the subsequent stages of a caterpillar infection, non-occluded virions are budded through the plasma membrane of the midgut cells into the haemolymph and hence propagate the infection to other cells and tissues of the insect. This systemic infection subsequently yields large quantities of occluded viruses that are important for the persistence of the baculovirus in the environment (hence subsequence infection cycles in other insect generations). Thus, the polyhedral inclusion body plays an important role in the natural infection of caterpillars by baculoviruses. This role is not emulated to the same efficiency by polyhedrin-negative viruses, as indicated by the reduced larval infections obtained using the non-occluded VC2 virus. Infection of *T.ni* caterpillars with the VC2 derived polyhedra produced a potent infection with the concomitment production of substantial amounts of LCMV-N protein.

The preparation of recombinant baculoviruses that produce polyhedrin and one (or more) foreign gene product simultaneously should circumvent some of the low infectivity problems associated with the use of polyhedrin-negative recombinant baculoviruses in the exploitation of caterpillars to produce large quantities of a protein product efficiently and cost effectively. Similarly the availability of genetically engineered baculoviruses with the capacity to express polyhedrin and a gene product toxic to caterpillars (or a gene that will produce other deleterious effects to the animal) may lead to the production of more efficacious baculovirus insecticides with a level of infectivity and persistence in the environment akin to that of the natural wild-type virus, allowing long-term pest protection (see Bishop, 1986). Based on the results described here the possibility of preparing and exploiting other multiple expression systems based on baculoviruses (or other vectors) is manifest, either by taking the route followed herein, or by placing the requisite regulatory gene machinery of the same or a genetically compatible organism (or virus) into other regions of a genome.

The plasmid replicon pAcVC2 has been modified by replacing the LCMV-N gene and polyhedrin gene with unique restriction enzyme sites that will allow any two foreign genes to be inserted and ultimately expressed simultaneously at the recombinant virus level. The derived general purpose replicon pAcVC3 with two PESs organised in non-overlapping sequences on opposite strands of DNA will allow foreign genes to be inserted into the plasmid by virtue of the unique *Bgl*II amd *Bam*HI restriction sequences. (See Fig. 8 and Example 1 (ii) MATERIALS AND METHODS (j) Construction of pAcVC3).

The hepatitis B (HB) virus sequences coding for the preC, or C antigens (HBpcAg, HBcAg) have been inserted into the baculovirus plasmid transfer vector, pAcYM1, such that the HB virus sequences are under the control of the polyhedrin promoter of *Autographa californica* nuclear polyhedrosis virus (AcNPV). *Spodoptera frugiperda* cells infected with either of the derived recombinant plasmids in the presence of infectious AcNPV DNA yielded recombinant, polyhedrin-negative, viruses that expressed high levels of the respective HBpcAg, or HBcAG (representing ca 5-10% [HBpcAg] and ca 40% [HBpcAg] of the stained cellular proteins respectively). The particulate 27 nm HBcAg have been purified to homogeneity from infected cell extracts by density gradient centrifugation. Dual expression transfer vectors containing the HBcAg gene sequences and the coding sequences of the HB virus S antigen (HBsAg), (each gene under its own copy of the polyhedrin promoter) have also been constructed and used to derive recombinant viruses. The recombinant with the HBVC and S genes expressed high levels of the HBcAg (*ca.* 40% of the cellular proteins) at the same time as low levels of the HBsAg (*ca* 2% of the stained cellular proteins). Dual expression, occluded, recombinant baculoviruses that make HBsAg as well as AcNPV polyhedrin protein have been prepared that are highly infectious for Trichoplusia ni caterpillars, allowing reproducible preparation of the antigen in a cost-effective manner.

Using radioimmunoassays (RIA) and ELISA, the recombinant HBcAg (RIA) and HBsAg (ELISA) have been used to identify human antibodies to HB virus with results that compare favourably with the data obtained with non-recombinant antigens.

Deposits

A sample of plasmic pAcVC3 in *E.coli* MC1061 has been deposited on 7th August 1987 under accession number NCIB12516 with the National Collection of Industrial and Marine Bacteria, 135 Abbey Road, P O Box 31, Aberdeen AB9 8DG.

A sample of recombinant virus VC4 has been deposited on 10th August 1987 under accession number 87081001 with the National Collection of Animal Cell Cultures PHLS, Centre for Applied Microbiology & Research, Porton Down, Salisbury, Wiltshire SP4 09G.

A sample of plasmid pAcYM1KTs in *E. coli* JM105 has been deposited on 25th July 1988 under accession number NCIB40033 with the National Collection of Industrial and Marine Bacteria, 135 Abbey Road, P O Box 31, Aberdeen AB9 8DG.

A sample of plasmid pAcVCKTs in *E. coli* JM105 has been deposited on 21st July 1988 under accession number NCIB40028 with the National Collection of Industrial and Marine Bacteria, 135 Abbey Road, P O Box 31, Aberdeen AB9 8DG.

A sample of plasmid pACVCKTsc in *E. coli* JM105 has been deposited on 21st July 1988 under accession number NCIB40029 with the National Collection of Industrial and Marine Bacteria, 135 Abbey Road, P O Box 31, Aberdeen AB9 8DG.

A sample of plasmid pAcVCKTspc in *E. coli* JM105 has been deposited on 21st July 1988 under accession number NCIB40030 with the National Collection of Industrial and Marine Bacteria, 135 Abbey Road, P O Box 31, Aberdeen AB9 8DG.

A sample of plasmid pAcYM1KTc in *E. coli* JM105 has been deposited on 21st July 1988 under accession number NCIB40031 with the National Collection of Industrial and Marine Bacteria, 135 Abbey Road, P O Box 31, Aberdeen AB9 8DG.

A sample of plasmid pAcYM1KTs in *E. coli* JM105 has been deposited on 21st July 1988 under accession number NCIB40032 with the National collection of Industrial and Marine Bacteria, 135 Abbey Road, P O Box 31, Aberdeen AB9 8DG.

TABLE 1. Anti-HBs levels in human sera tested by RIA and ELISA

|  |  | RIA [ELISA #1] | | | | |
|---|---|---|---|---|---|---|
|  |  | negative | <10 IU/L | 10-50 IU/L | 50-100 IU/L | >100 IU/L |
| ELISA #2 | negative | 5[90] |  |  |  |  |
|  | <10 IU/L | [1] | 1 |  |  |  |
|  | 10-50 IU/L |  |  | 5 |  |  |
|  | 50-100 IU/L |  |  |  | 3 |  |
|  | >100 IU/L |  |  |  | 1 | 72 |

Human antibody titers to HBsAg were determined using human plasma derived HBsAg for antibody capture (and detection) and reference anti-HBs from WHO in RIA assays, or from BPL (Elstree, UK) in [ELISA #1] assays. The data were compared to the human antibody titers determined using the recombinant HBsAg for antibody capture (and human plasma derived HBsAg for detection) and reference antibody from BPL in ELISA #2 assays.

TABLE 2. Anti-HBc analyses of human sera using RIA and liver or recombinant derived antigen

|  |  | RIA #1 | | |
|---|---|---|---|---|
|  |  | negative | weak positive | strong positive |
| RIA #2 | negative | 76 | 4 | 2 |
|  | weak positive | 2 |  |  |
|  | strong positive |  |  | 66 |

Human antibody titers to HBcAg were determined using liver (RIA #1) or recombinant derived HBcAg (RIA #2).

**Figure legends**

**Fig. 1.**    Schematic of the construction of the plasmid replicon pAcVC2.

**Fig. 2.**    Immunofluorescence of recombinant baculovirus-infected *S.frugiperda* cells. Cells were infected 36 h previously with AcNPV (**a, b**), or recombinant YM1.YN1 (Matsuura *et al.*, 1987; **c, d**), or the recombinant virus VC2 derived from recombinant replicon pAcVC2 (e.g.). In panels a,c and **e** the cells were fixed with acetone and examined for immunofluorescence using an LCMV-N specific monoclonal antibody (M. Buchmeier, Scripps Institute, La Jolla CA, USA). To observe polyhedral occlusion bodies, the same cells were examined by light microscopy (**b, d, g**). Cells infecteld with AcNPV contained polyhedral inclusion bodies (**b**). Cells infected with recombinant YM1.YN1 virus did not(**d**). Cells infected with recombinant VC2 showed both positive immunofluorescence (**e**) and polyhedral inclusion bodies (**g**). The hybrid ultraviolet/light micrograph of recombinant VC2 virus infected cells (**f**) clearly showed both immunofluorescence and polyhedra.

**Fig. 3.** Southern blot analyses of the recombinant baculovirus VC2 DNA. *Acc* II digests of VC2 viral DNA were recovered from AcNPV, the recombinant virus VC2 and from the recombinant replicon pAcVC2. The DNA products were resolved by agarose gel electrophoresis and probed with either nick-translated coding regions of polyhedrin DNA (upper panel), or LCMV-N DNA (lower panel). A 5082 nucleotide fragment in the *Acc* II digest of VC2 DNA was identified by both DNA probes corresponding to the *Acc* II fragment present in the recombinant replicon pAcVC2. As expected, the polyhedrin probe identified a 2028 nucleotide *Acc* II fragment in wild-type AcNPV, whilst the LCMV-N probe did not hybridise to any AcNPV DNA sequence.

**Fig. 4.** Northern blot analyses of total cellular RNA extracted from *S.frugiperda* cells infected with the recombinant virus VC2. The poly (A+) RNA species were recovered and processed as described in Methods. The blotted RNA was hybridised to nick-translated probes representing either the coding regions of the polyhedrin gene (left-hand panel), or the LCMV-N gene (right hand panel). *S.frugiperda* cells infected with wild-type AcNPV, or the recombinant virus YM1.YN1 that expresses LCMV-N protein were processed similarly and utilised as positive hybridisation controls. The polyhedrin probe hybridised to a 1.2 kb mRNA species in cellular RNA obtained from VC2 infected cells which corresponded to the polyhedrin mRNA species present in AcNPV infected cells. The LCMV-N probe hybridised to a 2.25 kb mRNA species in cellular RNA obtained from VC2 infected cells which corresponded to the LCMV-N mRNA species present in the recombinant YM1.YN1 virus infected cells.

**Fig. 5.** Metabolic labelling of proteins produced during infection with the recombinant baculorivus VC2. *S.frugiperda* cells were infected with virus VC2, or with wild-type AcNPV, or recombinant virus YM1.YN1 (containing only the LCMV-N gene). Proteins were labelled at 24 h and 48 h post-infection for 1 hr with [$^{35}$S]methionine, and the [$^{35}$S]-labelled proteins recovered, resolved by gel electrophoresis and fluorographed. Uninfected cells were treated similarly (U). The positions of the LCMV-N and AcNPV polyhedrin proteins are indicated.

**Fig. 6.** Expression of the LCMV-N and polyhedrin protein by the recombinant baculovirus VC2. Details of infection are equivalent to those described in the legend to the Fig. 5 except that the protein gel was stained with Kenacid Blue. The positions of the LCMV-N and polyhedrin proteins are indicated.

**Fig. 7.** Electron micrographs of uninfected or virus infected *S.frugiperda* cells. The panels show (**a**) uninfected *S.frugiperda*; (**b**) AcNPV infected *S.frugiperda*, illustrating polyhedral inclusion bodies in the nucleus; (**c**) *S.frugiperda* infected with recombinant YM1.YN1, note the dense cytoplasmic LCMV-N protein inclusion bodies; (**d**) and (**e**) *S.frugiperda* cells infected with recombinant virus VC2 exhibiting both intranuclear polyhedral inclusion bodies and cytoplasmic LCMV-N protein inclusion bodies.

**Fig. 8.** Schematic of the construction of the plasmid replicon pAcVC3.

**Fig. 9.** Schematic diagram of the construction of the transfer vectors pAcYM1KTpc and pAcYM1KTc as described in Methods. In the sequences shown at the sites of insertion the *Bam*HI site and the initiation codons of the HBpcAg and HBcAg are underlined.

**Fig. 10.** Schematic diagram of the construction of the transfer vector pAcYM1KTs as described in Methods. In the sequence shown the *Bam*HI site and the initiation codon of the HBsAg are underlined.

**Fig. 11.** Schematic diagram of the construction of the transfer vectors pAcVCKTspc and pAcVCKTsc as described in Methods.

**Fig. 12.** Schematic diagram of the construction of the transfer vector pAcVCKTs as described in Methods.

**Fig. 13.** The sequences of the HBcAg and HBpcAg genes and amino acids. The methionine codons are boxed that initiate the preC and C gene products. Open and filled triangles show nucleotide and amino acid differences, respectively, by comparison with sequences published by Ono and associates (1983).

**Fi.g. 14.**  Metabolic labelling (left) and Western blot analysis (right) of proteins expressed by recombinant baculoviruses. *S.frugiperda* cells were infected with recombinant viruses or wild-type AcNPV. Proteins were labelled at 48 h post-infection for 3 h with [$^{35}$S]methionine and the $^{35}$S-labelled protein recovered, resolved by gel electrophoresis, blotted to nitrocellulose membranes and fluorographed (left), or detected immunologically with anti-HBcAg serum (right). Uninfected cells were treated similarly (Mock). The positions of HBcAg (C), HBpcAg (pC), HBsAg (S), and AcNPV polyhedrin protein (P) are indicated. The positions of molecular weight marker proteins (12.5 - 92 kiloDaltons) are indicated on the left.

**Fi.g 15.**  Electron micrographs of HBCAg and HBsAg particles produced by *S. frugiperda* cells infected with recombinant viruses. (A) HBcAg was extracted by sonication from YM1KTc virus infected cells (alternatively, on occasion, by freeze-thawing the cells) and purified by CsCl isopicnic centrifugation as described in Methods, then stained with 1% uranyl acetate (B) HBsAg was collected from the supernatant fluids of YM1KTs virus infected cells, clarified by centrifugation (2,000xg) after treatment with 7% PEG, the antigen pelleted after adjusting the supernatant fluids with 9% PEG and stained with 1% uranyl acetate.

**Fig. 16.**  Kinetics of HBsAg synthesis from *S. frugiperda* cells infected with the recombinant YM1KTs (A) or VCKTsc (B). Infected cells (5 x $10^8$) were cultured in 500 ml media with stirring. Samples (5 ml) were collected every day and the media and cells separated by low speed centrifugation. Cells were lysed by sonication after gently rinsing with PBS. Viral HBsAg in the cell lysates, or in the clarified culture medium were measured by solid-phase RIA (AUSTRIA II, Abbott Laboratories) with the human derived HBsAg as a positive control and to quantitate the yields (see Methods). The data are expressed as the antigen per ml of original medium.

**Fi.g 17.**  Expression of HBcAg, HBpcAg, HBsAg and polyhedrin protein by recombinant baculoviruses. Cell extracts from recombinant or AcNPV infected cells were obtained at 2 days post-infection. After resolution by SDS-PAGE, the gel was stained with Kenacid Blue. The positions of the HBpcAg, HBcAg, HBsAg and polyhedrin protein are indicated.

**Fig. 18.**  Immunofluorescence of recombinant baculovirus-infected *S. frugiperda* cells. *S. frugiperda* cells infected 72 h previously with a recombinant baculovirus named VCKTsc were fixed with acetone and examined by indirect immunofluorescent assay as described in Methods, using (A) human anti-HBc serum, or (B) mouse monoclonal anti-HBs ascitic fluids. Uninfected *S. frugiperda* cells were treated similarly and examined using mouse monoclonal anti-HBs (C).

**Fig. 19.**  Electron micrographs of virus infected *S. frugiperda* cells. In (A) a thin section of a cell infected with recombinant VCKTsc is shown, in (B) another cell at higher magnification. The filled triangles point to the massed positions of HBcAg.

**Claims**

1.  A plasmid replicon for use in introducing a plurality of genes into an expression vector, said plasmid replicon comprising double-stranded DNA containing sequences (a), (b1), (b2) and (c) defined as follows:

    (a) a sequence allowing the replicon to be reproduced in a bacterial host,

    (b1 and b2) first and second sequences which are adapted to permit an intervening sequence located between said first and second sequences to be introduced into an expression vector, and

    (c) an intervening sequence located between said first and second sequences (b1) and (b2),

    characterised in that the intervening sequence (c) comprises first and second polypeptide expression sequences (PESs) designated (c1) and (c2),

    wherein said first PES (c1) includes (i) a first transcriptional promotor, (ii) a first unique restriction site for first introduction of a first gene which is native or foreign to the expression vector, said first gene being under the control of said first transcriptional promotor, and (iii) a first transcriptional termination site, and said second PES (c2) includes (iv) a second transcriptional promotor, (v) a second unique restriction site for introduction of a second gene which is native or foreign to the expression vector, said second gene being under the control of said second transcriptional promotor, and (vi) a second transcriptional termination site.

2.  A plasmid replicon according to Claim 1 wherein the first and second PESs are arranged in the opposite sense to one another on separate strands of the DNA.

3. A plasmid replicon according to Claim 1 wherein the first and second PESs are arranged in the same sense on the same strand of DNA.

4. A plasmid replicon according to Claim 3 wherein a selectable gene or an essential functional gene for the expression vector is located between the two PESs.

5. A plasmid replicon according to any preceding claim wherein each PES includes a different unique restriction site for introduction of a gene which is native or foreign to the expression vector.

6. A plasmid replicon according to any preceding claim including a plurality of pairs of said first and second PESs.

7. A plasmid replicon for use in introducing a plurality of genes into an expression vector, said plasmid replicon comprising double-stranded DNA containing sequences (a), (b1), (b2) and (c) defined as follows:
   (a) a sequence allowing the replicon to be reproduced in a bacterial host,
   (b1 and b2) first and second sequences which are adapted to permit an intervening sequence located between said first and second sequences to be introduced into an expression vector, and
   (c) an intervening sequence located between said first and second sequences (b1) and (b2),
   characterised in that the intervening sequence (c) comprises first and second polypeptide expression sequences (PESs) designated (c1) and (c2),
   wherein said first PES (c1) includes (i) a first transcriptional promotor, (ii) a first gene which is native or foreign to the expression vector, said first gene being under the control of said first transcriptional promotor , and (iii) a first transcriptional termination site, and said second PES (c2) includes (iv) a second transcriptional promotor, (v) a second gene which is native or foreign to the expression vector said sec ond gene being under the control of said second transcriptional promoter, and (vi) a second transcriptional termination site.

8. A plasmid replicon according to Claim 7 wherein the first and second PESs are arranged in the opposite sense to one another on separate strands of the DNA.

9. A plasmid replicon according to Claim 7 wherein the first and second PESs are arranged in the same sense on the same strand of DNA.

10. A plasmid replicon according to any of Claims 7 to 9 wherein a selectable gene or an essential functional gene for the expression vector is located between the two PESs.

11. A plasmid replicon according to any of Claims 7 to 10 wherein one of said PESs includes a structural gene which codes for AcNPV polyhedrin protein.

12. A plasmid replicon according to any of Claims 7 to 11 wherein one of said PESs includes at least an antigenic portion of hepatitis B surface antigen.

13. A plasmid replicon according to any of Claims 7 to 12 wherein one of said PESs includes at least an antigenic portion of hepatitis B core antigen.

14. A plasmid replicon according to any of Claims 7 to 10 wherein one of said PESs includes a structural gene which codes for at least an antigenic portion of hepatitis B surface antigen and another of said PESs codes for at least an antigenic portion of hepatitis B core antigen.

15. A sequence cassette for use in constructing a plasmid replicon as claimed in any of Claims 1 to 14, said sequence cassette comprising double-stranded DNA containing first and second polypeptide expression sequences (PESs) designated (c1) and (c2),
   wherein said first PES (c1) includes (i) a first transcriptional promotor, (ii) a first unique restriction site for introduction of a first gene which is native or foreign to the expression vector, said first gene being under the control of said first transcriptional promoter, and (iii) a first transcriptional termination site, and said second PES (c2) includes (iv) a second transcriptional promotor, (v) a second unique restriction site for introduction of a second gene which is native or foreign to the expression vector, said second gene being under the control of said second transcriptional promoter and (vi) a second transcriptional termination site,

and wherein said PESs are flanked by flanking sequences which are homologous with sequences of an expression vector, such that when inserted in the vector, no essential functional genes of the expression vector are lost.

16. A sequence cassette according to Claim 15 wherein the arms are homologous to sequences of the vector genome, which sequences are arranged so as to allow the intervening sequences to be introduced without loss of vector DNA sequences.

17. A sequence cassette according to Claim 15 wherein the arms are homologous to sequences of the vector genome, which sequences are arranged so as to allow the intervening sequences to be introduced with replacement of inessential vector DNA sequences.

18. A sequence cassette according to Claim 15 wherein the arms are homologous to sequences of the vector genome, which sequences are arranged so as to allow the intervening sequences to be introduced so as to replace intergenic or non-regulatory regions of the vector genome.

19. A set of sequence cassettes, each according to any of Claims 15 to 18, wherein the homologous regions of each cassette of the set are homologous to different regions of the vector genome, allowing a plurality of pairs of PESs to be introduced into the vector genome, with each pair being located at a separate location.

20. A viral expression vector, suitable for transfecting an insect cell and having an insert adapted to direct synthesis in the cell of at least one polypeptide not normally encoded by nuclear DNA of the cell, said insert comprising double stranded DNA containing a sequence which comprises first and second polypeptide expression sequences (PESs) designated (c1) and (c2),

wherein said first PES (c1) includes (i) a first transcriptional promotor, (ii) a first gene which is native or foreign to the expression vector said first gene being under the control of said first transcriptional promoter, and (iii) a first transcriptional termination site, and said second PES (c2) includes (iv) a second transcriptional promotor, (v) a second gene which is native or foreign to the expression vector, said second gene being under the control of said second transcriptional promoter and (vi) a second transcriptional termination site.

21. A viral expression vector according to Claim 20 wherein one of said structural genes codes for a viral protein normally expressed during infection of the insect cells by wild type virus.

22. A viral expression vector according to Claim 20 or Claim 21 wherein at least one of said promotors is a promotor for a viral protein normally expressed during infection of the insect cells by wild type virus.

23. A viral expression vector according to Claim 22 wherein the insect virus is a baculovirus.

24. A viral expression vector according to any of Claims 20 to 23 wherein at least one of said promotors is the promotor of the polyhedrin gene of *Autographa californica* nuclear polyhedrosis virus.

25. A viral expression vector according to any of Claims 20 to 24 wherein one of said PESs includes a structural gene which codes for AcNPV polyhedrin protein.

26. A viral expression vector according to any of Claims 20 to 25 wherein one of said PESs includes at least an antigenic portion of hepatitis B surface antigen.

27. A viral expression vector according to any of Claims 20 to 26 wherein one of said PESs includes at least an antigenic portion of hepatitis B core antigen.

28. A viral expression vector according to any of Claims 20 to 24 wherein one of said PESs includes a structural gene which codes for at least an antigenic portion of hepatitis B surface antigen and another of said PESs codes for at least an antigenic portion of hepatitis B core antigen.

**29.** An expression vector according to any of Claims 20 to 28 produced by introducing into a suitable recipient vector a sequence cassette as claimed in any of Claims 15 to 18 or a set of sequence cassettes as claimed in Claim 19.

**30.** A method of constructing a viral expression vector suitable for transfecting an insect cell and having an insert adapted to direct synthesis in the cell of at least one polypeptide not normally encoded by nuclear DNA of the cell, said insert comprising double-stranded DNA having an intervening sequence located between first and second sequences, said intervening sequence comprising first and second polypeptide expression sequences (PESs), wherein each PES includes (i) a transcriptional promoter, (ii) a gene which is native or foreign to the expression vector, and (iii) a transcriptional termination site, which comprises the step of utilizing a plasmid replicon as claimed in any of Claims 7 to 14 to transform a vector lacking said insert into a vector possessing said insert.

**31.** A process for producing one or more desired polypeptides, which comprises the step of infecting susceptible insects or insect cells with a viral expression vector suitable for transfecting an insect cell and having an insert adapted to direct synthesis in the cell of at least one polypeptide not normally encoded by nuclear DNA of the cell, said insert comprising double-stranded DNA containing a sequence which comprises first and second polypeptide expression sequences (PESs) designated (c1) and (c2),
wherein said first PES (c1) includes (i) a first transcriptional promotor, (ii) a first gene which is native or foreign to the expression vector said first gene being under the control of said first transcriptional promoter, and (iii) a first transcriptional termination site, and said second PES (c2) includes (iv) a second transcriptional promotor, (v) a second gene which is native or foreign to the expression vector, said second gene being under the control of said second transcriptional promoter and (vi) a second transcriptional termination site.

**32.** A process according to Claim 31 wherein said process involves infecting individuals of an insect species capable of being infected by the virus.

**33.** A process according to Claim 32 wherein the individuals are in the form of caterpillars.

**Patentansprüche**

**1.** Plasmidreplikon zur Verwendung in der Einschleusung einer Mehrzahl von Genen in einen Expressionsvektor, wobei das Plasmidreplikon doppelsträngige DNA umfaßt, die die nachstehend definierten Sequenzen (a), (b1), (b2) und (c) enthält:
(a) eine Sequenz, welche die Neubildung des Replikons in einem Bakterienwirt erlaubt,
(b1 und b2) erste und zweite Sequenzen, die die Einschleusung einer zwischen den ersten und den zweiten Sequenzen befindlichen Zwischensequenz in einen Expressionsvektor zu erlauben, und
(c) eine Zwischensequenz, die sich zwischen den ersten und zweiten Sequenzen (b1) und (b2) befindet,
dadurch gekennzeichnet, daß die Zwischensequenz (c) mit (c1) und (c2) bezeichnete erste und zweite Polypeptidexpressionssequenzen (PESen) umfaßt,
wobei die erste PES (c1) (i) einen ersten Transkriptionspromotor, (ii) eine erste einmal vorkommende Restriktionsspaltstelle für die erste Einschleusung eines ersten Gens, das ursprünglich in dem Expressionsvektor vorkommt oder für diesen fremd ist, wobei das erste Gen sich unter der Kontrolle des ersten Transkriptionspromotors befindet, und (iii) eine erste Transkriptionsterminationsstelle enthält, und die zweite PES (c2) (iv) einen zweiten Transkriptionspromotor, (v) eine zweite einmal vorkommende Restriktionsspaltstelle für die Einschleusung eines zweiten Gens, das ursprünglich in dem Expressionsvektor vorkommt oder für diesen fremd ist, wobei das zweite Gen sich unter der Kontrolle des zweiten Transkriptionspromotors befindet, und (vi) eine zweite Transkriptionsterminationsstelle enthält.

**2.** Plasmidreplikon nach Anspruch 1, wobei die ersten und zweiten PESen auf getrennten DNA-Strängen im gegenläufigen Sinn zueinander angeordnet sind.

**3.** Plasmidreplikon nach Anspruch 1, wobei die ersten und zweiten PESen im selben Sinn auf dem selben DNA-Strang angeordnet sind.

4. Plasmidreplikon nach Anspruch 3, wobei sich ein selektierbares Gen oder ein für den Expressionsvektor essentielles funktionelles Gen zwischen den beiden PESen befindet.

5. Plasmidreplikon nach einem der vorstehenden Ansprüche, wobei jede PES eine unterschiedliche einmal vorkommende Restriktionsspaltstelle für die Einschleusung eines Gens, das ursprünglich in dem Expressionsvektor vorkommt oder für diesen fremd ist, enthält.

6. Plasmidreplikon nach einem der vorstehenden Ansprüche, das eine Mehrzahl von Paaren der ersten und zweiten PESen enthält.

7. Plasmidreplikon zur Verwendung in der Einschleusung einer Mehrzahl von Genen in einen Expressionsvektor, wobei das Plasmidreplikon doppelsträngige DNA umfaßt, die die nachstehend definierten Sequenzen (a), (b1), (b2) und (c) enthält:
   (a) eine Sequenz, welche die Neubildung des Replikons in einem Bakterienwirt erlaubt,
   (b1 und b2) erste und zweite Sequenzen, die die Einschleusung einer zwischen den ersten und den zweiten Sequenzen befindlichen Zwischensequenz in einen Expressionsvektor erlauben, und
   (c) eine Zwischensequenz, die sich zwischen den ersten und zweiten Sequenzen (b1) und (b2) befindet,
   dadurch gekennzeichnet, daß die Zwischensequenz (c) mit (c1) und (c2) bezeichnete erste und zweite Polypeptidexpressionssequenzen (PESen) umfaßt,
   wobei die erste PES (c1) (i) einen ersten Transkriptionspromotor, (ii) ein erstes Gen, das ursprünglich in dem Expressionsvektor vorkommt oder für diesen fremd ist, wobei das erste Gen sich unter der Kontrolle des ersten Transkriptionspromotors befindet, und (iii) eine erste Transkriptionsterminationsstelle enthält, und die zweite PES (c2) (iv) einen zweiten Transkriptionspromotor, (v) ein zweites Gen, das ursprünglich in dem Expressionsvektor vorkommt oder für diesen fremd ist, wobei das zweite Gen sich unter der Kontrolle des zweiten Transkriptionspromotors befindet, und (vi) eine zweite Transkriptionsterminationsstelle enthält.

8. Plasmidreplikon nach Anspruch 7, wobei die ersten und zweiten PESen auf getrennten DNA-Strängen im gegenläufigen Sinn zueinander angeordnet sind.

9. Plasmidreplikon nach Anspruch 7, wobei die ersten und zweiten PESen im selben Sinn auf dem selben DNA-Strang angeordnet sind.

10. Plasmidreplikon nach Anspruch 7 oder 9, wobei sich ein selektierbares Gen oder ein für den Expressionsvektor essentielles funktionelles Gen zwischen den beiden PESen befindet.

11. Plasmidreplikon nach einem der Ansprüche 7 bis 10, wobei eine der PESen ein AcNPV-Polyhedrinprotein codierendes Strukturgen enthält.

12. Plasmidreplikon nach einem der Ansprüche 7 bis 11, wobei eine der PESen mindestens einen antigenen Teil des Hepatitis B-Oberflächenantigens enthält.

13. Plasmidreplikon nach einem der Ansprüche 7 bis 12, wobei eine der PESen mindestens einen antigenen Teil des Hepatitis B-Kernantigens enthält.

14. Plasmidreplikon nach einem der Ansprüche 7 bis 10, wobei eine der PESen ein Strukturgen enthält, das mindestens einen antigenen Teil des Hepatitis B-Oberflächenantigens codiert und eine andere der PESen mindestens einen antigenen Teil des Hepatitis B-Kernantigens codiert.

15. Sequenzcassette zur Verwendung in der Konstruktion eines Plasmidreplikons nach einem der Ansprüche 1 bis 14, wobei die Sequenzcassette doppelsträngige DNA umfaßt, die mit (c1) und (c2) bezeichnete erste und zweite Polypeptidexpressionssequenzen (PESen) enthält, und
   wobei die erste PES (c1) (i) einen ersten Transkriptionspromotor, (ii) eine erste einmal vorkommende Restriktionsspaltstelle für die Einschleusung eines ersten Gens, das ursprünglich in dem Expressionsvektor vorkommt oder für diesen fremd ist, wobei das erste Gen sich unter der Kontrolle des ersten Transkriptionspromotors befindet, und (iii) eine erste Transkriptionsterminationsstelle enthält und die zweite PES (c2) (iv) einen zweiten Transkriptionspromotor, (v) eine zweite einmal vorkommende

EP 0 327 626 B1

Restriktionsspaltstelle für die Einschleusung eines zweiten Gens, das ursprünglich in dem Expressionsvektor vorkommt oder für diesen fremd ist, wobei das zweite Gen sich unter der Kontrolle des zweiten Transkriptionspromotors befindet, und (vi) eine zweite Transkriptionsterminationsstelle enthält, und wobei die PESen von mit Sequenzen eines Expressionsvektors homologen flankierenden Sequenzen flankiert sind, so daß bei Einfügung in den Vektor keine essentiellen funktionellen Gene des Expressionsvektors verloren gehen.

16. Sequenzcassette nach Anspruch 15, wobei die Arme zu Sequenzen des Vektorgenoms homolog sind und die Sequenzen so angeordnet sind, daß sie die Einschleusung der Zwischensequenzen ohne Verlust von Vektor-DNA-Sequenzen erlauben.

17. Sequenzcassette nach Anspruch 15, wobei die Arme zu Sequenzen des Vektorgenoms homolog sind und die Sequenzen so angeordnet sind, daß sie die Einschleusung der Zwischensequenzen unter Ersatz von nicht-essentiellen Vektor-DNA-Sequenzen erlauben.

18. Sequenzcassette nach Anspruch 15, wobei die Arme zu Sequenzen des Vektorgenoms homolog sind und die Sequenzen so angeordnet sind, daß sie die Einschleusung der Zwischensequenzen unter Ersatz von intergenen oder nicht-Regulations-Bereichen des Vektorgenoms erlauben.

19. Satz von Sequenzcassetten, jeweils nach einem der Ansprüche 15 bis 18, wobei die homologen Bereiche jeder Cassette des Satzes zu verschiedenen Bereichen des Vektorgenoms homolog sind, wodurch eine Mehrzahl von PESen-Paaren in das Vektorgenom eingeschleust werden kann und wobei sich jedes Paar an einem anderen Ort befindet.

20. Viraler Expressionsvektor, der zur Transfektion einer Insektenzelle geeignet ist und eine Insertion aufweist, die zur Steuerung der Synthese mindestens eines normalerweise von nuclearer DNA der Zelle nicht codierten Polypeptids in der Zelle geeignet ist, wobei die Insertion doppelsträngige DNA umfaßt, die eine Sequenz enthält, welche mit (c1) und (c2) bezeichnete erste und zweite Polypeptidexpressionssequenzen (PESen) umfaßt, wobei die erste PES (c1) (i) einen ersten Transkriptionspromotor, (ii) ein erstes Gen, das ursprünglich in dem Expressionsvektor vorkommt oder für diesen fremd ist, wobei das erste Gen sich unter der Kontrolle des ersten Transkriptionspromotors befindet, und (iii) eine erste Transkriptionsterminationsstelle enthält, und die zweite PES (c2) (iv) einen zweiten Transkriptionspromotor, (v) ein zweites Gen, das ursprünglich in dem Expressionsvektor vorkommt oder für diesen fremd ist, wobei das zweite Gen sich unter der Kontrolle des zweiten Transkriptionspromotors befindet, und (vi) eine zweite Transkriptionsterminationsstelle enthält.

21. Viraler Expressionsvektor nach Anspruch 20, wobei eines der Strukturgene ein virales Protein codiert, das normalerweise vom Wildtyp-Virus während der Infektion der Insektenzelle exprimiert wird.

22. Viraler Expressionsvektor nach Anspruch 20 oder 21, wobei mindestens einer der Promotoren ein Promotor für ein virales Protein ist, das normalerweise vom Wildtyp-Virus während der Infektion der Insektenzelle exprimiert wird.

23. Viraler Expressionsvektor nach Anspruch 22, wobei das Insektenvirus ein Baculovirus ist.

24. Viraler Expressionsvektor nach einem der Ansprüche 20 bis 23, wobei mindestens einer der Promotoren der Promotor des Polyhedringens des nuclearen Autographa californica Polyhedrosisvirus ist.

25. Viraler Expressionsvektor nach einem der Ansprüche 20 bis 24, wobei eine der PESen ein Strukturgen enthält, das AcNPV Polyhedrinprotein codiert.

26. Viraler Expressionsvektor nach einem der Ansprüche 20 bis 25, wobei eine der PESen mindestens einen antigenen Teil des Hepatitis B-Oberflächenantigens enthält.

27. Viraler Expressionsvektor nach einem der Ansprüche 20 bis 26, wobei eine der PESen mindestens einen antigenen Teil des Hepatitis B-Kernantigens enthält.

27

**28.** Viraler Expressionsvektor nach einem der Ansprüche 20 bis 24, wobei eine der PESen ein Strukturgen enthält, das mindestens einen antigenen Teil des Hepatitis B-Oberflächenantigens codiert und eine andere der PESen mindestens einen antigenen Teil des Hepatitis B-Kernantigens codiert.

**29.** Expressionsvektor nach einem der Ansprüche 20 bis 28, hergestellt durch Einschleusung einer Sequenzcassette nach einem der Ansprüche 15 bis 18 oder eines Satzes Sequenzcassetten nach Anspruch 19 in einen geeigneten aufnehmenden Vektor.

**30.** Verfahren zur Konstruktion eines viralen Expressionsvektors, der zur Transfektion einer Insektenzelle geeignet ist und eine Insertion aufweist, die zur Steuerung der Synthese mindestens eines normalerweise von nuclearer DNA der Zelle nicht codierten Polypeptids in der Zelle geeignet ist, wobei die Insertion doppelsträngige DNA umfaßt, die eine zwischen ersten und zweiten Sequenzen befindliche Zwischensequenz aufweist, und wobei die Zwischensequenz erste und zweite Polypeptidexpressionssequenzen (PESen) umfaßt, wobei jede PES (i) einen Transkriptionspromotor, (ii) ein Gen, das ursprünglich in dem Expressionsvektor vorkommt oder für diesen fremd ist, und (iii) eine Transkriptionsterminationsstelle enthält, das den Schritt der Verwendung eines Plasmidreplikons nach einem der Ansprüche 7 bis 14 zur Umwandlung eines Vektors, der diese Insertion nicht aufweist, in einen diese Insertion aufweisenden Vektor umfaßt.

**31.** Verfahren zur Herstellung von einem oder mehreren gewünschten Polypeptiden, das den Schritt der Infektion geeigneter Insekten oder Insektenzellen mit einem viralen Expressionsvektor umfaßt, der zur Transfektion einer Insektenzelle geeignet ist und eine Insertion aufweist, die zur Steuerung der Synthese mindestens eines normalerweise von nuclearer DNA der Zelle nicht codierten Polypeptids in der Zelle geeignet ist, wobei die Insertion doppelsträngige DNA umfaßt, die eine Sequenz enthält, welche mit (c1) und (c2) bezeichnete erste und zweite Polypeptidexpressionssequenzen (PESen) umfaßt,
wobei die erste PES (c1) (i) einen ersten Transkriptionspromotor, (ii) ein erstes Gen, das ursprünglich in dem Expressionsvektor vorkommt oder für diesen fremd ist, wobei das erste Gen sich unter der Kontrolle des ersten Transkriptionspromotors befindet, und (iii) eine erste Transkriptionsterminationsstelle enthält, und die zweite PES (c2) (iv) einen zweiten Transkriptionspromotor, (v) ein zweites Gen, das ursprünglich in dem Expressionsvektor vorkommt oder für diesen fremd ist, wobei das zweite Gen sich unter der Kontrolle des zweiten Transkriptionspromotors befindet, und (vi) eine zweite Transkriptionsterminationsstelle enthält.

**32.** Verfahren nach Anspruch 31, wobei das Verfahren die Infektion von Tieren einer Insektenart einschließt, die von dem Virus infiziert werden kann.

**33.** Verfahren nach Anspruch 32, wobei die Tiere sich im Raupenstadium befinden.

**Revendications**

**1.** Réplicon de plasmide servant à introduire plusieurs gènes dans un vecteur d'expression, ce réplicon de plasmide comprenant un ADN bicaténaire contenant les séquences (a), (b1) (b2) et (c) définies de la façon suivante :

(a) est une séquence permettant au réplicon d'être reproduit dans un hôte bactérien,
(b1) et (b2) sont des première et secondes séquences qui sont adaptées pour permettre l'introduction d'une séquence intermédiaire située entre ces première et seconde séquences dans un vecteur d'expression, et
(c) est une séquence intermédiare située entre ces première et seconde séquences (b1) et (b2),
caractérisé en ce que la séquence intermédiaire (c) comprend des première et seconde séquences d'expression de polypeptide (PES) désignées (c1) et (c2),
dans laquelle cette première PES (c1) comprend : (i) un premier promoteur transcriptionnel, (ii) un premier site de restriction unique pour une première introduction d'un premier gène qui est naturel ou étranger au vecteur d'expression, ce premier gène étant sous le contrôle de ce premier promoteur transcriptionnel, et (iii) un premier site de terminaison transcriptionnel, et cette seconde PES (c2) comprend : (iv) un second promoteur transcriptionnel, (v) un seconde site de restriction unique pour l'introduction d'un second gène qui est naturel ou étranger au vecteur d'expression, ce seconde gène étant sous le contrôle de ce second promoteur transcriptionnel, et (vi) un second site de terminaision

28

de transcription.

2. Réplicon de plasmide suivant la revendication 1, dans lequel les première et seconde PES sont disposées en sens opposées l'une del'autre sur des brins séparés de l'ADN.

3. Réplicon de plasmide suivant la revendication 1, dans lequel les première et seconde PES sont disposées dans le même sens sur le même brin d'ADN.

4. Réplicon de plasmide suivant la revendication 3, dans lequel un gène pouvant être sélectionné ou un gène fonctionnel essentiel pour le vecteur d'expression, est situé entre les deux PES.

5. Réplicon de plasmide suivant l'une quelconque des revendications précédentes dans laquelle chaque PES comprend un site de restriction unique différent pour l'introduction d'un gène qui est naturel ou étranger au vecteur d'expression.

6. Réplicon de plasmide suivant l'une quelconque des revendications précédentes, comprenant plusieurs paires de ces première et seconde PES.

7. Réplicon de plasmide servant à introduire plusieurs gènes dans un vecteur d'expression, ce réplicon de plasmide comprenant un ADN bicaténaire contenant les séquences (a), (b1) (b2) et (c) définies de la façon suivante :
   (a) est une séquence permettant au réplicon d'être reproduit dans un hôte bactérien,
   (b1) et (b2) sont des première et secondes séquences qui sont adaptées pour permettre l'introduction d'une séquence intermédiare située entre ces première et seconde séquences dans un vecteur d'expression, et
   (c) est une séquence intermédiaire située entre ces première et seconde séquences (b1) et (b2),
   caractérise en ce que la séquence intermédiaire (c) comprend des première et seconde séquences d'expression de polypeptide (PES) désignées (c1) et (c2),
   dans laquelle cette première PES (c1) comprend : (i) un premier promoteur transcriptionnel, (ii) un premier gène est naturel ou étranger au vecteur d'expression, ce premier gène étant sous le contrôle de ce premier promoteur transcriptionnel, et (iii) un premier site de terminaison de transcription, et cette seconde PES (c2) comprend : (iv) un second promoteur transcriptionnel, (v) un second gène qui est naturel ou étranger au vecteur d'expression, ce second gène étant sous le contrôle de ce second promoteur transcriptionnel, et (vi) un second site de terminaison de transcription.

8. Réplicon de plasmide suivant la revendication 7, dans lequel les première et seconde PES sont disposées en sens opposée l'une de l'autre sur des brins séparés de l'ADN.

9. Réplicon de plasmide suivant la revendication 7, dans lequel les première et seconde PES sont disposées dans le même sens sur le même brin d'ADN.

10. Réplicon de plasmide suivante l'une quelconque des revendications 7 à 9, dans lequel un gène pouvant être sélectionée ou un gène fonctionnel essentiel pour le vecteur d'expression, est situé entre les deux PES.

11. Réplicon de plasmide suivant l'une quelconque des revendications 7 à 10, dans lequel l'une de ces PES comprend un gène structural qui code pour la protéine polyhédrine AcNPV.

12. Réplicon de plasmide suivante l'une quelconque des revendications 7 à 11, dans lequel l'une de ces PES comprend au moins une partie antigénique d'antigène de surface de l'hépatite B.

13. Réplicon de plasmide suivant l'une quelconque des revendications 7 à 12, dans lequel l'une de ces PES comprend au moins une partie antigénique d'antigène de noyau de l'hépatite B.

14. Réplicon de plasmide suivant l'une quelconque des revendications 7 à 10, dans lequel l'une de ces PES comprend un gène structural qui code pour au moins une partie antigénique d'antigène de surface de l'hépatite B et l'autre de ces PES code pour au moins une partie antigénique d'antigène de noyau de l'hépatite B.

**15.** Cassette de séquences servant à la construction d'un réplicon de plasmide suivant l'une quelconque des revendications 1 à 14, cette cassette de séquences comprenant un ADN bicaténaire contenant des première et seconde séquences d'expression de polypeptide (PES) désignées (c1) et (c2),

dans laquelle cette première PES (c1) comprend : (i) un premier promoteur transcriptionnel, (ii) un premier site de restriction unique pour l'introduction d'un premier gène qui est naturel ou étranger au vecteur d'expression, ce premier gène étant sous le contrôle de ce premier promoteur transcriptionnel, et (iii) un premier site de terminaison de transcription, et cette seconde PES (c2) comprend : (iv) un second promoteur transcriptionnel, (v) un second site de restriction unique pour l'introduction d'un second gène qui est naturel ou étranger au vecteur d'expression, ce second gène étant sous le contrôle de ce second promoteur transcriptionnel, et (vi) un second site de terminaison de transcription, et dans laquelle ces PES sont flanquées de séquences flanquantes qui sont homologues de séquences d'un vecteur d'expression, de telle sorte que lorsqu'elles sont insérées dans le vecteur, il n'est perdu aucun gène fonctionnel essentiel du vecteur d'expression.

**16.** Cassette de séquences suivant la revendication 15, dans laquelle les bras sont homologues de séquences du génome du vecteur, lesquelles séquences sont arrangées de façon à permettre l'introduction des séquences intermédiares sans perte de séquences d'ADN du vecteur.

**17.** Cassette de séquences suivant la revendication 15, dans laquelle les bras sont homologues de séquences du génome du vecteur, lesquelles séquences sont arrangées de façon à permettre l'introduction des séquences intermédiaires avec remplacement de séquences non essentielles d'ADN du vecteur.

**18.** Cassette de séquences suivant la revendication 15, dans laquelle les bras sont homologues de séquences du génome du vecteur, lesquelles séquences sont arrangées de façon à permettre l'introduction des séquences intermédiaires de façon à remplacer des régions intergéniques ou non régulatrices du génome du vecteur.

**19.** Série de cassettes de séquences, chacune suivant l'une quelconque des revendications 15 à 18, dans laquelle les régions homologues de chacune cassette de la série sont homologues de différentes régions de génome de vecteur, permettant l'introduction de plusieurs paires de PES dans le génome de vecteur, chaque paire étant située dans une situation séparée.

**20.** Vecteur d'expression virale, convenable pour transfecter une cellule d'insecte et ayant un insert adapté pour diriger la synthèse dans la cellule d'au moins un polypeptide qui n'est pas normalement codé par l'ADN nucléaire de la cellule, cet insert comprenant un ADN bicaténaire contenant une séquence qui comprend des première et seconde séquences d'expression de polypeptide (PES) désignées (c1) et (c2),

dans laquelle cette première PES (c1) comprend : (i) un premier promoteur transcriptionnel, (ii) un premier gène qui est naturel ou étranger au vecteur d'expression, ce premier gène étant sous le contrôle de ce premier promoteur transcriptionnel, et (iii) un premier site de terminaison de transcription, et cette seconde PES (c2) comprend : (iv) un second promoteur transcriptionnel, (v) un second gène est naturel ou étranger au vecteur d'expression, ce second gène étant sous le contrôle de ce second promoteur transcriptionnel, et (vi) un second site de terminaison de transcription.

**21.** Vecteur d'expression virale suivant la revendication 20, dans lequel l'un de ces gènes structuraux code pour une protéine virale normalement exprimée pendant l'infection des cellules d'insecte par un virus de type sauvage.

**22.** Vecteur d'expression virale suivant les revendications 20 ou 21, dans lequel l'un au moins de ces promoteurs est un promoteur pour une protéine virale normalement exprimée pendant l'infection des cellules d'insecte par un virus de type sauvage.

**23.** Vecteur d'expression virale suivant la revendication 22, dans lequel le virus d'insecte est un baculovirus.

**24.** Vecteur d'expression virale suivant l'une quelconque des revendications 20 à 23, dans lequel l'un au moins de ces promoteurs est le promoteur du gène de la polyhédrine du virus de la polyhédrose nucléaire *Autographa californica.*

25. Vecteur d'expression virale suivant l'une quelconque des revendications 20 à 24, dans lequel l'une de ces PES comprend un gène structural qui code pour la protéine polyhédrine AcNPV.

26. Vecteur d'expression virale suivante l'une quelconque des revendications 20 à 25, dans lequel l'une de ces PES comprend au moins une partie antigénique d'antigène de surface de l'hépatite B.

27. Vecteur d'expression virale suivant l'une quelconque des revendications 20 à 26, dans lequel l'une de ces PES comprend au moins une partie antigénique d'antigène de noyau de l'hépatite B.

28. Vecteur d'expression virale suivant l'une quelconque des revendications 20 à 24, dans lequel l'une de ces PES comprend un gène structural qui code pour au moins une partie antigénique d'antigène de surface de l'hépatite B et l'autre de ces PES code pour au moins une partie antigénique d'antigène de noyau de l'hépatite B.

29. Vecteur d'expression suivant l'une quelconque des revendications 20 à 28, produit par introduction dans un vecteur récepteur convenable d'une cassette de séquences suivant l'une quelconque des revendications 15 à 18 ou une série de cassettes suivant la revendication 19.

30. Méthode pour construire un vecteur d'expression virale convenable pour transfecter une cellule d'insecte et ayant un insert adapté pour diriger la synthèse dans la cellule d'au moins un polypeptide qui n'est pas normalement codé par l'ADN nucléaire de la cellule, cet insert comprenant un ADN bicaténaire ayant une séquence intermédiaire située entre des première et seconde séquences d'expression cette séquence intermédiaire comprenant des première et seconde séquences d'expression de polypeptide (PES), dans laquelle chaque PES comprend : (i) un promoteur transcriptionnel, (ii) un gène qui est naturel ou étranger au vecteur d'expression et (iii) un site de terminaison de transcription, qui comprend l'étape d'utilisation d'un réplicon de plasmide suivant l'une quelconque des revendications 7 à 14 pour transformer un vecteur manquant de cet insert en un vecteur possédant cet insert.

31. Procédé pour produire un ou plusieurs polypeptides désirés, qui comprend l'étape d'infection d'insectes ou de cellules d'insectes sensibles avec un vecteur d'expression virale convenable pour transfecter une cellule d'insecte et ayant un insert adapté pour diriger la synthèse dans la cellule d'au moins un polypeptide qui n'est pas normalement codé par l'ADN nucléaire de la cellule, cet insert comprenant un ADN bicaténaire contenant une séquence qui comprend des première et seconde séquences d'expression de polypeptide (PES) désignées (c1) et (c2),
dans laquelle cette première PES (c1) comprend : (i) un premier promoteur transcriptionnel, (ii) un premier gène qui est naturel ou étranger au vecteur d'expression, ce premier gène étant sous le contrôle de ce premier promoteur transcriptionnel, et (iii) un premier site de terminaison de transcription, et cette seconde PES (c2) comprend : (iv) un second promoteur transcriptionnel, (v) un second gène qui est naturel ou étranger au vecteur d'expression, ce second gène étant sous le contrôle de ce second promoteur transcriptionnel, et (vi) un second site de terminaison de transcription.

32. Procédé suivant la revendication 31, dans lequel ce procédé implique l'infection d'individus d'une espèce d'insecte susceptible d'être infectée par le virus.

33. Procédé suivant la revendication 32, dans lequel les individus sont sous la forme de chenilles.

FIG.1

FIG. 2

**Polyhedrin probe**

**LCM-N probe**

AcNPV  VC2  pAcVC2

AcNPV  VC2  pAcVC2

◀ 5.080kb

2.028kb ▶

◀ 5.080kb

*FIG.3*

**Poly(A⁺)mRNA**

VC2  AcNPV  YM1YN1

VC2  AcNPV  YM1YN1

1.2kb ▶

◀ 2.25kb

**Polyhedrin Probe**

**LCM-N Probe**

*FIG.4*

34

U    AcNPV    VC2    YM1YN1

24h    48h    24h    48h    24h    48h

◀ LCM-N protein

Polyhedrin ▶

*FIG.5*

AcNPV    VC2    YM1YN1
24h    48h    24h    48h    24h    48h

◀ LCM-N protein

Polyhedrin ▶

*FIG.6*

35

FIG.7

FIG.8

FIG.9

pSCK102

HinPl  Styl
HBcAg
Styl
HinPl
pBR322

HinPl digestion

Styl digestion

BamHl cut

pAcYM1

pUC8
Klenow fill in
dephosphorylation

Klenow fill in

BamHl cut

pAcYM1

pUC8
Klenow fill in
dephosphorylation

Klenow fill in

ligation
Bgl II

EcoRV

pAcYM1KTpc

pUC8

ligation
BlgII

EcoRV

pAcYM1KTc

pUC8

EcoRV-Bgl II
fragment
sequencing

EcoRV-Bgl II
fragment
sequencing

...AAATACGGATCCGCACCAGCACCATGCAA...

...AAATACGGATCCTTGGGTGGCTTTGGGGCATGGAC...

construction of expression vectors to produce HBcAG

... AAATACGGATCCGAGGACTGGGGACCCTGTGACGAACATGGAG...

*FIG.10*

Construction of an expression vector to produce HBsAg

Construction of multiple expression vectors to produce HBsAg and HBcAg.

FIG.11

Construction of a multiple expression vector to produce HBsAg and Polyhedrin

*FIG.12*

41

EP 0 327 626 B1

preCore ┐

Core ┐

HinP1

StyI

```
          M  Q  L  F  H  L  C  L  I  I  S  C  T  C  P  T  V  Q  A  S  K  L  C  L  G  W  L  W  G  M  D  I  D  P  Y  K
GCGCACCAGCACCATGCAACTTTTTCACCTCTGCCTAATCATCTCTTGTACATGTCCCACTGTTCAAGCCTCCAAGCTGTGCCTTGGGTGGCTTTGGGGCATGGACATTGACCCTTATAA
     1810       1820       1830       1840       1850       1860       1870       1880       1890       1900       1910       1920


    E  F  G  A  T  V  E  L  L  S  F  L  P  S  D  F  F  P  S  V  R  D  L  L  D  T  A  S  A  L  Y  R  E  A  L  E  S  P  E  H
AGAATTTGGAGCTACTGTGGAGTTACTCTCGTTTTTGCCTTCTGACTTTTTTCCTTCCGTCAGAGATCTCCTAGACACCGCCTCAGCTCTGTATCGGGAAGCCTTAGAGTCTCCTGAGCA
     1930       1940       1950       1960       1970       1980       1990       2000       2010       2020       2030       2040


    C  S  P  H  H  T  A  L  R  Q  A  I  L  C  W  G  E  L  M  T  L  A  T  W  V  G  N  N  L  E  D  P  A  S  R  D  L  V  V  N,
TTGCTCACCTCACCATACTGCACTCAGGCAAGCAATTCTCTGCTGGGGGGAATTGATGACTCTAGCTACCTGGGTGGGTAATAATTTGGAAGATCCAGCATCCAGGGATCTAGTAGTCAA
     2050       2060       2070       2080       2090       2100       2110       2120       2130       2140       2150       2160


    Y  V  N  T  N  M  G  L  K  I  R  Q  L  L  W  F  H  I  S  C  L  T  F  G  R  E  T  V  L  E  Y  L  V  S  F  G  V  W  I  R
TTATGTTAATACTAACATGGGTTTAAAGATCAGGCAACTATTGIGGTTTCATATATCTTGCCTTACTTTTGGAAGAGAGACTGTACTTGAATATTTGGTCTCTTTCGGAGTGTGGATTCG
     2170       2180       2190       2200       2210       2220       2230       2240       2250       2260       2270       2280


    T  P  P  A  Y  R  P  P  N  A  P  I  L  S  T  L  P  E  T  T  V  V  R  R  R  D  R  G  R  S  P  R  R  R  T  P  S  P  R  R
CACTCCTCCAGCCTATAGACCACCAAATGCCCCTATCTTATCAACACTTCCGGAAACTACTGTTGTTAGACGACGGGACCGAGGCAGGTCCCCTAGAAGAAGAACTCCCTCGCCTCGCAG
     2290       2300       2310       2320       2330       2340       2350       2360       2370       2380       2390       2400


    R  R  S  Q  S  P  R  R  R  R  S  Q  S  R  E  S  Q  C  *
ACGCAGATCTCAATCGCCGCGTCGCAGAAGATCTCAATCTCGGGAATCTCAATGTTAGTA
     2410       2420       2430       2440       2450       2460
```

*FIG.13*

FIG.14

A) HBcAg

B) HBsAg

FIG.15

A) YM1KTs          B) VCKTsc

cell culture medium
cell lysate
total

HBsAg (ng/ml)

500

250

0

Days postinfection          Days postinfection

FIG.16

EP 0 327 626 B1

FIG.17

FIG.18

FIG.19